# EUROPÄISCHE PATENTSCHRIFT

(11) **EP 3 342 463 B1**
(45) Veröffentlichungstag und Bekanntmachung des Hinweises auf die Patenterteilung: **25.09.2019**
(21) Anmeldenummer: 18155862.8
(22) Anmeldetag: 05.03.2013
(51) Int. Cl.: A61N 5/10

(54) **VERFAHREN UND BESTRAHLUNGSANLAGE ZUR BESTRAHLUNG EINES ZIELVOLUMENS**
METHOD AND IRRADIATION INSTALLATION FOR IRRADIATING A TARGET VOLUME
PROCÉDÉ ET INSTALLATION D'IRRADIATION D'UN VOLUME CIBLE

(30) Priorität: 05.03.2012 DE 102012004170
(43) Veröffentlichungstag der Anmeldung: 04.07.2018
(62) Teilanmeldung aus: 13709358.9
(73) Patentinhaber: GSI Helmholtzzentrum für Schwerionenforschung GmbH, 64291 Darmstadt (DE)
(72) Erfinder: BERT, Christoph, 91080 Uttenreuth (DE); GEMMEL, Alexander, 91060 Erlangen (DE); LÜCHTENBORG, Robert, 64287 Darmstadt (DE)
(74) Vertreter: Blumbach · Zinngrebe Patentanwälte PartG mbB

(56) Entgegenhaltungen:
- EP-A1- 2 400 506
- SCHULTE R ET AL: "Design of a proton computed tomography system for applications in proton radiation therapy", 2003 IEEE NUCLEAR SCIENCE SYMPOSIUM CONFERENCE RECORD. / 2003 IEEE NUCLEAR SCIENCE SYMPOSIUM AND MEDICAL IMAGING CONFERENCE. PORTLAND, OR, OCT. 19 - 25, 2003; [IEEE NUCLEAR SCIENCE SYMPOSIUM CONFERENCE RECORD], NEW YORK, NY : IEEE, US, 19. Oktober 2003 (2003-10-19), Seite 1579, XP010735912, ISBN: 978-0-7803-8257-2
- "SISTEMA AQUA "ADVANCED QUALITY ASSURANCE" PER IL CENTRO NAZIONALE DI ADROTERAPIA ONCOLOGICA", , 15. Februar 2008 (2008-02-15), Seiten 1-134, XP055005634, Gefunden im Internet: URL:http://project-aqua.web.cern.ch/projec t-aqua/RESOURCES/CNAO_TERA_REPORT.pdf [gefunden am 2011-08-25]

## Beschreibung

### Gebiet der Erfindung

Die Erfindung betrifft ein Verfahren und eine Bestrahlungsanlage zur Bestrahlung eines Zielvolumens mit einem lonenstrahl, insbesondere für die Tumortherapie.

### Hintergrund der Erfindung

Eine Bewegung des Tumors in der lonenstrahltherapie stellt besondere Herausforderungen an die Bestrahlung, um sicherzustellen, dass das klinische Zielvolumen trotz der Bewegung mit der verschriebenen Dosis abgedeckt wird. Für die lonenstrahltherapie mit einem aufgestreuten Strahl werden hierfür typischerweise speziell konstruierte Sicherheitssäume verwendet, was bislang als ausreichend angesehen wurde.

Bei einem gescannten Strahl kommt es allerdings zu Interferenzeffekten, die weitergehende Maßnahmen erfordern. Unter diesen sind Strahlapplikation mittels sogenanntem Gating oder sogenanntem Beam Tracking, die jeweils auf einem Bewegungserfassungssystem basieren, welches die Tumorbewegung oder ein Surrogat der Tumorbewegung liefern, das in Echtzeit verwendet wird, um den Strahl ggf. zu unterbrechen (Gating) oder aktiv nachzuführen (Beam Tracking). Der Güte dieses Signals kommt eine wesentliche Rolle zu, da die Präzision direkten Einfluss auf die Präzision der gesamten Bestrahlung hat. Gating und Beam Tracking sind dem Fachmann grundsätzlich bekannt; vgl. zum Gating z.B. "Respiratory Gated Irradiation System for Heavy-Ion Radiotherapy" von Shinichi Minohara et al. in Int. J. Oncology Biol. Phys., Vol. 47, No. 4, pp. 1097-1103, 2000 oder "Gated Irradiation with Scanned Particle Beams von Christoph Bert et al. in Int. J. Oncology Biol. Phys., Vol. 73, No. 4, pp. 1270-1275, 2009 und zum Beam Tracking z.B. DE 10 2004 028 035 A1.

Es existieren diverse Bewegungserfassungssysteme auf dem Markt. Manche Bewegungserfassungssysteme messen ein sogenanntes Bewegungssurrogat, beispielsweise die Atemtemperatur, die Bewegung der Bauchdecke (in 1D, 2D oder 3D), den Umfang des Abdomens/Thorax oder den Fluss der Atemluft. Andere Bewegungserfassungssysteme erfassen im Gegensatz dazu direkt die Bewegung des Tumors und basieren z.B. auf Fluoroskopie (mit/ohne implantierten radioopaquen Markern), Radiotranspondern oder Ultraschall. Außerdem werden auch Kombinationen dieser Systeme verwendet, um deren Vorteile zu kombinieren, beispielsweise die spärliche Fluoroskopie (hohe Güte, aber Dosisbelastung für den Patienten) neuronal vernetzt mit einem Bauchdeckenerfassungssystem (niedrigere Güte, aber keine Dosisbelastung für den Patienten).

Bewegungserfassungssysteme zur direkten Erfassung der Tumorbewegung gehen häufig einher mit einem chirurgischen Eingriff in den Patienten oder mit einer deutlich erhöhten Dosisbelastung des Patienten, z.B. bei der konventionellen Röntgenfluoroskopie, insbesondere bei Verwendung von hohen Bilderfassungsraten. Externe Surrogat-basierte Bewegungserfassungssysteme sind prinzipbedingt darauf beschränkt eine sehr indirekte Messung durchzuführen, weshalb u.a. die Präzision der Erfassung der Tumorbewegung verbesserungswürdig ist. Ferner kann die Tumorbewegung, z.B. bei einem Lungentumor hochkomplex sein und jeweils translatorische, rotatorische und kompressorische/dilatatorische in allen Dimensionen Komponenten enthalten.

Alle genannten Systeme betrachten darüber hinaus die rein geometrische Bewegung des Zielvolumens, die in der Ionentherapie nur in Verbindung z.B. mit 4DCT-Datensätzen verwendet wird, da die Reichweite des Strahls von wesentlicher Bedeutung ist. Für die präzise Deposition der gewünschten Dosis bei der Bestrahlung ist nämlich nicht ausschließlich die räumliche Bewegung maßgeblich, sondern vielmehr die Wirkung der räumlichen Bewegung auf den Energieverlust des lonenstrahls, der von anderen Faktoren, wie z.B. der lokalen Verteilung der Gewebedichte abhängen kann.

Daher sind diese bekannten Verfahren insbesondere im Hinblick auf die Präzision weiter verbesserungswürdig.

Aus der EP 2 400 506 ist eine Vorrichtung bekannt, welche zumindest zwei verschiedene Partikelstrahlen erzeugt, von denen der zweite Partikelstrahl zur Detektion der Bewegung des Zielvolumens verwendet wird. Hierzu werden zwei Ionenquellen zur Erzeugung unterschiedlicher lonentypen verwendet, die in einer Mischkammer zusammengeführt werden.

Dieses Verfahren lässt sich allerdings nur für bestimmte Kombinationen von Ionen verwenden und hängt von der Art der Beschleunigereinrichtung ab. Ferner sind bestimmte Parameter der beiden lonenstrahlen nur gemeinsam beeinflussbar. Daher wurde von den Erfindern, insbesondere hinsichtlich der Komplexität, Flexibilität und der Beschränkungen durch die Verschränkung der beiden lonenstrahlen bei der in der EP 2 400 506 beschriebenen Herangehensweise hier nach einer anderen Lösung gesucht.

### Allgemeine Beschreibung der Erfindung

Die Erfindung hat sich daher die Aufgabe gestellt, ein Verfahren und eine Bestrahlungsanlage zur Bestrahlung eines Zielvolumens mit einem lonenstrahl bereit zu stellen, welche eine hohe Präzision der Bestrahlung trotz eines sich bewegenden Zielvolumens ermöglichen.

Ein weiterer Aspekt der Aufgabe der Erfindung ist es, ein Verfahren und eine Bestrahlungsanlage zur Bestrahlung eines sich bewegenden Zielvolumens mit einem lonenstrahl bereit zu stellen, welche die nachteilige Wirkung der Bewegung des Zielvolumens auf den Energieverlust und die Reichweite des lonenstrahls in dem Zielvolumen möglichst präzise ermittelt werden kann.

Noch ein Aspekt der Aufgabe der Erfindung ist es, ein Verfahren und eine Bestrahlungsanlage zur Bestrahlung eines sich bewegenden Zielvolumens mit einem lonenstrahl bereit zu stellen, welche eine präzise, aber dennoch flexible Bewegungserfassung des Zielvolumens ermöglicht, die möglichst unabhängig von der Beschleunigereinrichtung und ggf. sogar einfach nachrüstbar ist.

Die Aufgabe der Erfindung wird durch den Gegenstand der unabhängigen Ansprüche gelöst. Vorteilhafte Weiterbildungen der Erfindung sind in den Unteransprüchen definiert.

Es wird ein Verfahren zur Bestrahlung eines sich während der Bestrahlung bewegenden Zielvolumens mit einem lonenstrahl bereit gestellt. Der lonenstrahl wird zunächst von einer Beschleunigereinrichtung erzeugt und beschleunigt und zum Zielvolumen geführt. Die Beschleunigereinrichtung umfasst insbesondere einen Kreisbeschleuniger wie ein Zyklotron oder Synchrotron, einen Linearbeschleuniger oder eine Kombination hieraus.

Unter dem Begriff lonenstrahl wird insbesondere ein Protonenstrahl oder ein Strahl aus schwereren Ionen wie z.B. Sauerstoff oder Kohlenstoff verstanden. Derartige Bestrahlungsanlagen aus der Entwicklung der beiden Anmelderinnen finden sich z.B. bei der GSI Helmholtzzentrum für Schwerionenforschung GmbH in Darmstadt oder beim Heidelberger lonenstrahl-Therapiezentrum (HIT), wo insbesondere mit ¹²C-Ionen bestrahlt wird. Es sollen aber auch andere geladene hadronische Partikelstrahlen, wie Pionen etc. nicht ausgeschlossen sein.

Erfindungsgemäß wird nun die Bestrahlung des Zielvolumens zeitlich in zumindest eine Radiographiephase und zumindest eine Depositionsphase unterteilt, um die Energie des lonenstrahls, d.h. ein- und desselben lonenstrahls, zwischen der zumindest einen Radiographiephase und der zumindest einen Depositionsphase zeitlich zu variieren wird und zwar derart, dass
i) in der zumindest einen Radiographiephase die Reichweite des lonenstrahls distal des Zielvolumens (in Strahlrichtung hinter dem Zielvolumen) liegt, so dass in der zumindest einen Radiographiephase der lonenstrahl das Zielvolumen durchdringt oder durchleuchtet, um mittels des lonenstrahls ein Ionenradiogramm des Zielvolumens aufzunehmen, indem der das Zielvolumen durchdringende lonenstrahl mit einem distal des Zielvolumens angeordneten Ionenradiographiedetektor detektiert wird und
ii) in der zumindest einen Depositionsphase die Reichweite des lonenstrahls innerhalb des Zielvolumens liegt, so dass der lonenstrahl in dem Zielvolumen abgestoppt wird, um eine vorbestimmte, nämlich die in dem Bestrahlungsplan geplante Dosis in dem Zielvolumen zu deponieren.

Mittels der Ionenradiographie kann die Bewegung des Zielvolumens erfasst werden, wobei aber die Radiographie und die Deposition mit ein- und demselben lonenstrahl, aber mit unterschiedlicher Energie und zeitlich konsekutiv durchgeführt werden. In der Radiographiephase wird die Strahlreichweite demnach so eingestellt, dass das Bragg-Maximum distal des Patienten, genauer in dem Ionenradiographiedetektor liegt, um die Lage des Bragg-Maximums mittels des energie- und ortsauflösenden Ionenradiographiedetektors zu messen, indem der lonenstrahl in dem Ionenradiographiedetektor abgestoppt wird. Hierfür wird die Energie des lonenstrahls also in der Radiographiephase auf eine höhere Radiographieenergie und während der Depositionsphase auf eine niedrigere Depositionsenergie eingestellt. Der Wechsel zwischen der Radiographiephase und der Depositionsphase besteht demnach im Umschalten der Energie des lonenstrahls.

Beispielsweise wird ein Kohlenstoff-Ionenstrahl erzeugt, welcher bei einer Energie E' im Bereich von 600 MeV/u das Zielvolumen im Wesentlichen vollständig durchdringt und mit dieser Energie E' zur Radiographie und mit einer herabgesetzt auf eine Energie E im Bereich von 250 MeV/u zur Dosisdeposition gemäß dem Bestrahlungsplan verwendet wird.

Erfindungsgemäß kann also mit ein und demselben lonenstrahl sowohl die Dosisdeposition als auch die Radiographie durchgeführt werden und es ist hinreichend, lediglich die Energie zu variieren bzw. "umzuschalten". Die Variierung bzw. Umschaltung der Strahlenergie kann hierbei während der Bestrahlung, insbesondere in Echtzeit, z.B. innerhalb eines sogenannten Spills bei einer Synchroton-basierten Beschleunigereinrichtung oder in einer Bestrahlungspause zwischen den Spills erfolgen. Das Variieren oder Umschalten zwischen der Depositionsenergie und der Radiographieenergie ist nicht zu verwechseln mit der weitaus geringeren Änderung der Energie zur Bestrahlung verschiedener Isoenergieschichten, bei welcher die Strahlreichweite lediglich um einige Millimeter verändert wird und welche ggf. zusätzlich durchgeführt wird. In vorteilhafter Weise können aufgrund der zeitlichen Trennung bestimmte Parameter der Deposition und der Ionenradiographie unabhängig voneinander eingestellt werden, insbesondere die laterale Abdeckung des Zielvolumens bei einem Scannverfahren.

In vorteilhafter Weise ist mit der Erfindung eine hochpräzise Bewegungsverfolgung möglich. Aus den Ionenradiogrammen lassen sich Reichweiteänderungen, verursacht durch die Bewegung des Zielvolumens direkt ermitteln ohne eine fehlerbehaftete Umrechnung aus der Röntgenabschwächung in die Änderung der Teilchenreichweite zu benutzen. In dem Ionenradiogramm wird die Bewegung des Zielvolumens nämlich in gleicher Weise "gesehen" wie bei der Deposition, da der Energieverlust desselben lonenstrahls ermittelt wird. Der einzige Unterschied zwischen der Deposition und der Radiographie ist die Energie des lonenstrahls, was die Unterschiede in der Wirkung der Bewegung (translatorisch, rotatorisch, Kompression/Dilatation) minimiert. Mit anderen Worten wird bei der Ionenradiographie dieselbe physikalische Wirkung der Bewegung des Zielvolumens gemessen, die auch bei der Deposition auftritt - bis auf die unterschiedliche Ionenenergie, deren Einfluss aber sehr genau berechnet werden kann. Ferner ist im Vergleich zu konventioneller Fluoroskopie mit einer niedrigeren Dosis zu rechnen.

In vorteilhafter Weise kann das Verfahren trotzdem ohne größere Umbauten der Beschleunigereinrichtung durchgeführt werden. Besonders einfach wird z.B. die Beschleunigereinrichtung auf die höhere Radiographieenergie eingestellt und wird dann mittels eines passiven Energiemodulators, in der Depositionsphase oder den Depositionsphasen von der höheren Radiographieenergie auf die niedrigere Depositionsenergie herabgesetzt, indem der lonenstrahl in dem Energiemodulator abgebremst wird. Hiermit kann die Energie des lonenstrahls in einfacher Weise schnell genug variiert werden, um abwechselnd den Tumor zu bestrahlen (Deposition) und die Radiographie durchzuführen sowie in Echtzeit in Ansprechen auf die Bewegung des Zielvolumens steuernd in die Bestrahlung eingreifen zu können. Z.B. wird hierfür ein digitaler, z.B. ein sich drehender tortenförmiger Energiemodulator proximal des Zielvolumens (in Strahlrichtung vor dem Zielvolumen) durchstrahlt, bei dem ein Tortenstück fehlt, um die Energie und damit die Reichweite des Strahls zwischen einer Position im Zielvolumen und einer Position distal des Patienten zu modulieren. Im Bereich des fehlenden Tortenstücks bleibt die Energie des lonenstrahls unverändert (Radiographiephase) und im übrigen Bereich wird die Energie des lonenstrahls durch Abbremsung in der Materie des Modulators auf die Depositionsenergie herabgesetzt. Dieser insbesondere digitale Modulator ist wiederum nicht zu verwechseln mit den bekannten Keilsystemen zum aktiven Nachführen der Strahlreichweite an die Bewegung des Zielvolumens beim Beam Tracking, welche ggf. zusätzlich vorhanden sind.

Alternativ kann die Energie durch binäre Modulatorplatten verändert werden, wie z.B. in "The PSI Gantry 2: a second generation proton scanning gantry" von Eros Pedroni et al. in Z. Med. Phys. 14 (2004), 25-34 beschrieben ist oder durch eine Variation der Einstellungen auf dem Fiat-Top bei Synchrotron-basierter Beschleunigung, wie z.B. in "Update of an Accelerator Control System for the New Treatment Facility at HIMAC" von Y. Iwata et al. in Proceedings of EPAC08, Genoa, Italy, pp. 1800-1802 beschrieben ist, welche hiermit diesbezüglich durch Referenz inkorporiert werden. Allerdings ist es auch möglich, einen sogenannten Cyc-LINAC zu verwenden, wie z.B. in "High Frequency Linacs for Hadrontherapy" von Ugo Amaldi et al. in Reviews of Accelerator Science and Technology, Vol. 2 (2009), 111-131 beschrieben ist, welche hiermit diesbezüglich durch Referenz inkorporiert wird. Bei dem Cyc-LINAC können Reichweitensprünge in der Größenordnung wie sie zum Umschalten zwischen der Radiographieenergie und der Depositionsenergie notwendig sind, durch Ausschalten und Anschalten von einzelnen Kavitäten des Linearbeschleunigers erfolgen. Die Variation der Energie wird demnach vorzugsweise erst nach der Beschleunigung in der Beschleunigereinrichtung (passiver Modulator) oder zum Ende der Beschleunigung (Cyc-LINAC) vorgenommen, jedenfalls vorzugsweise nicht vor der Beschleunigung.

Insbesondere ist der Ionenradiographiedetektor demnach ein energieauflösender Detektor, welcher die (Rest-)Energie des lonenstrahls nach dem Durchdringen des Zielvolumens misst. Hiermit kann der Energieverlust, verursacht durch das Durchdringen des Zielvolumens berechnet werden und hieraus wiederum die Wirkung der Bewegung des Zielvolumens auf die Deposition ermittelt werden.

Insbesondere wird also ein- und derselbe lonenstrahl für die Deposition und die Ionenradiographie in dem Sinne verwendet, dass die lonensorte und die Ladung identisch sind und lediglich die Energie unterschiedlich ist. Es wird also ein- und derselbe lonenstrahl verwendet, dessen Energie zeitlich konsekutiv variiert wird, nicht jedoch gleichzeitig zwei verschiedene lonenstrahlen.

Mit anderen Worten werden die Ionenradiographie und die Deposition zeitlich nacheinander und im Rahmen dieser Voraussetzung unabhängig voneinander durchgeführt. Insbesondere wird in der Radiographiephase nicht deponiert und/oder in der Depositionsphase nicht Ionen-radiographiert.

Gemäß einer bevorzugten Ausführungsform der Erfindung wird die Bestrahlung des Zielvolumens zeitlich in eine Mehrzahl von Radiographiephasen und eine Mehrzahl von Depositionsphasen unterteilt,
wobei die Energie des lonenstrahls, insbesondere während der Bestrahlung oder in einer Bestrahlungspause, zwischen den Radiographiephasen und den Depositionsphasen wechselweise hin und her geschaltet wird, derart, dass zyklisch abwechselnd:
i) in den Radiographiephasen die Reichweite des lonenstrahls distal des Zielvolumens liegt, so dass in den Radiographiephasen der lonenstrahl das Zielvolumen durchdringt oder durchleuchtet und mittels des lonenstrahls Ionenradiogramme des Zielvolumens aufgenommen werden, indem der lonenstrahl mit einem distal des Zielvolumens angeordneten Ionenradiographiedetektor detektiert wird und
ii) in den Depositionsphasen die Reichweite des lonenstrahls in dem Zielvolumen liegt, so dass der lonenstrahl in dem Zielvolumen abgestoppt wird, um jeweils eine vorbestimmte Dosis in dem Zielvolumen zu deponieren.

In vorteilhafter Weise kann die zeitliche Abfolge der Radiographiephasen an die Bewegungsphasen des Zielvolumens angepasst werden. Alternativ oder zusätzlich kann die zeitliche Abfolge der Radiographiephasen an die Extraktionsphasen der Beschleunigereinrichtung (z.B. Spills bei einem Synchrotron) und/oder an den Ablauf der Bestrahlung der Isoenergieschichten angepasst werden, wenn das Zielvolumen in Isoenergieschichten unterteilt ist, die sukzessive bestrahlt werden.

Wenn in den Depositionsphasen verschiedene Isoenergieschichten des Zielvolumens mit dem lonenstrahl angefahren werden, um jeweils eine vorbestimmte Dosis in den Isoenergieschichten zu deponieren, kann z.B. zumindest vor der Bestrahlung zur Dosisdeposition jeder Isoenergieschicht eine Radiographiemessung gemäß i) durchgeführt wird, und/oder es wird zu Beginn jeder Bewegungsphase oder jedes Spills eine lonen-Radiographiemessung durchgeführt.

Vorzugsweise wird die Intensität des lonenstrahls in der zumindest einen oder der Mehrzahl von Depositionsphasen erheblich höher eingestellt, als in der zumindest einen oder der Mehrzahl von Radiographiephasen, was ebenfalls in Echtzeit gesteuert werden kann. Hiermit kann in vorteilhafter Weise die Dosisbelastung des Patienten niedrig gehalten werden.

Wenn das Zielvolumen ein sich während der Bestrahlung zyklisch bewegendes Zielvolumen ist, z.B. ein Lungentumor bei der Atmung, wird die zyklische Bewegung des Zielvolumens in mehrere Bewegungsphasen unterteilt. In Verbindung mit der Erfindung ist es vorteilhaft, die Zeitdauer der zumindest einen Radiographiephase oder der Mehrzahl von Radiographiephasen jeweils nicht länger zu wählen als die Zeitdauer der Bewegungsphasen. Dann kann, falls gewünscht, in - vorzugsweise zu Beginn - jeder Bewegungsphase eine lonen-Radiographiemessung durchgeführt werden. Mit anderen Worten werden die vorstehend definierten Schritte i) und ii) in derselben Bewegungsphase durchgeführt bzw. die Radiographiephase und Depositionsphase liegen zumindest teilweise in derselben Bewegungsphase.

In vorteilhafter Weise kann hiermit eine besonders präzise und zuverlässige Bewegungsverfolgung erzielt werden.

Besonders bevorzugt wird die Erfindung mit einer Bestrahlung mit aktiver Nachführung des lonenstrahls zur Kompensation der Bewegung des Zielvolumens (sogenanntes Beam Tracking) kombiniert. Vorzugsweise wird das aktive Nachführen des lonenstrahls, d.h. das Beam Tracking, in Ansprechen auf die mittels des Ionenradiographiedetektors durchgeführte Ionen-Radiographiemessung gesteuert. Diese Steuerung des aktiven Nachführens des lonenstrahls (Beam Tracking) in Ansprechen auf die Radiographiemessung kann ebenfalls in Echtzeit durchgeführt werden.

In vorteilhafter Weise kann aufgrund der zeitlichen Trennung der Radiographie und der Deposition der lonenstrahl in der Radiographiephase und in der Depositionsphase unabhängig voneinander gesteuert werden. Insbesondere kann der lonenstrahl in der Radiographiephase, unabhängig von der Deposition, über die laterale Ausdehnung des Zielvolumens gesteuert werden.

Erfindungsgemäß ist der Ionenradiographiedetektor als ortsauflösender Detektor ausgebildet, so dass in der zumindest einen oder der Mehrzahl von Radiographiephasen ein lateral zweidimensional ortsaufgelöstes Ionenradiogramm, vorzugsweise zumindest von Teilen des internen Zielvolumens (Internal Target Volume, ITV, gemäß ICRU 62) aufgenommen wird, indem eine Vielzahl von Rasterpunkten des Zielvolumens durchdrungen werden und die Reichweite des lonenstrahls nach dem Durchdringen des Zielvolumens für jeden der Rasterpunkte in dem Ionenradiogramm ermittelt wird, um eine zumindest zweidimensionale Karte der (Wasseräquivalent-)Reichweite des lonenstrahls zu erstellen. Diese Karte der Reichweite des lonenstrahls kann z.B. als Monitoring- oder Steuerinformation für zumindest eine nachfolgende Depositionsphase verwendet werden.

Eine zumindest zweidimensionale Darstellung des Zielvolumens im Ionenradiogramm ermöglicht in vorteilhafter Weise eine besonders präzise und zuverlässige Verfolgung der Bewegung des Zielvolumens.

Wenn das Bestrahlungsverfahren ein Scann-Verfahren, z.B. ein Raster-Scann-Verfahren ist, wird der lonenstrahl als sogenannter Bleistiftstrahl (Pencil-Beam) in der zumindest einen Depositionsphase oder der Mehrzahl von Depositionsphasen zumindest über einen Teil des klinischen Zielvolumens gescannt und in der zumindest einen Radiographiephase oder der Mehrzahl von Radiographiephasen zumindest über einen Teil der lateralen Fläche des Zielvolumens gewobbelt.

In vorteilhafter Weise kann hiermit trotz der Verwendung eines feinen Bleistiftstrahls ein lateral zweidimensionales Ionenradiogramm aufgenommen werden. Ferner ist von Vorteil, dass das Wobbeln zur Radiographiemessung unabhängig von dem Scannen bei der Deposition durchgeführt werden kann.

Das ist insbesondere weiter vorteilhaft, da Reichweitenverluste - falls gewünscht - feiner als auf Rasterpunktbasis der Deposition vorberechnet werden können. Dies hat z.B. für das Wobbeln, bei dem der Strahl schnell über einen größeren Bereich gefahren wird, den Vorteil, dass mehr oder sogar alle Positionen direkt verglichen werden können, ohne auf nominelle Rasterpositionen beschränkt zu sein. Eine feinere Auflösung kann z.B. in der Größenordnung von der CT-Voxelgröße sein. Die CT-Voxelgröße beträgt z.B. lediglich etwa 1 mm, während der Abstand der Rasterpunkte bei der Bestrahlung mit dem Scann-Verfahren typischerweise im Bereich von 2 bis 3 mm beträgt.

Beim Scann-Verfahren wird in der zumindest einen oder der Mehrzahl von Depositionsphasen der lonenstrahl über das klinische Zielvolumen (sogenanntes Clinical Target Volume, CTV gemäß ICRU 50) gescannt. Bevorzugt wird in der zumindest einen oder der Mehrzahl von Radiographiephasen der lonenstrahl zumindest über einen über das klinische Zielvolumen hinausgehenden Teil der lateralen Fläche des internen Zielvolumens (ITV, gemäß ICRU 62) gewobbelt.

Durch ein solches schnelles Wobbeln wird die laterale Position des Strahls über alle Areale des internen Zielvolumens (ITV) - und damit über die integrale Ausdehnung des klinischen Zielvolumens in allen Bewegungsphasen - gefahren. Dieses Wobbeln des gesamten internen Zielvolumens kann in einem Zeitintervall zwischen 1 ms und 1000 ms, beispielsweise im Bereich von 10 ms, 50 ms, 100 ms oder 500 ms erfolgen, so dass in dieser Zeit ein lateral zweidimensionales Radiogramm des internen Zielvolumens aufgenommen werden kann.

Wenn mit anderen Worten bei der Radiographiemessung ein größeres Areal bestrahlt wird, als das klinische Zielvolumen (CTV), insbesondere wenn zumindest das interne Zielvolumen (ITV) abgedeckt wird, das das klinischen Zielvolumen (CTV) in allen Bewegungszuständen abbildet, kann in vorteilhafter Weise die gesamte Bewegungsamplitude des Zielvolumens abgedeckt werden.

Ferner kann eine Reichweiten-Simulationsrechnung durchgeführt werden, um simulierte Sollwerte für die Reichweite des lonenstrahls zu berechnen. Während der Bestrahlung in der Radiographiephase wird dann die tatsächliche (Wasseräquivalent-)Reichweite des lonenstrahls nach dem Durchdringen des Zielvolumens ermittelt und die ermittelten tatsächlichen Reichweiten werden mit den simulierten Sollwerten verglichen.

Es ist insbesondere vorteilhaft, die Reichweiten-Simulationsrechnung für eine Vielzahl von Rasterpunkten durchzuführen und eine mehrdimensionale simulierte Sollwert-Karte (sogenannte "Range Map") der Reichweite des lonenstrahls zu erstellen. Währens der Bestrahlung in der Radiographiephase wird dann die tatsächliche Reichweite des lonenstrahls nach dem Durchdringen des Zielvolumens ebenfalls für eine Vielzahl von Rasterpunkten ermittelt und hieraus ein mehrdimensionales Ionenradiogramm mit den jeweiligen tatsächlichen Reichweiten des lonenstrahls erstellt und das Ionenradiogramm wird mit der simulierten Sollwert-Karte verglichen.

Durch die Vorberechnung einer simulierten Reichweitenkarte, einer sogenannten Range Map, insbesondere einer sogenannten Digital Rekonstruierten Range Map (DRRM) und durch den entsprechenden Vergleich zwischen Messung und Simulationsrechnung kann dabei in vorteilhafter Weise ein Abgleich zwischen der Bewegung des Zielvolumens und der Bewegung des lonenstrahls hergestellt werden, bei dem potentiell nicht nur Parameter in Bezug auf die Bewegung und Reichweiteänderung akquiriert werden können, sondern auch über die Interferenz zwischen beiden, insbesondere das Interplay oder Muster.

Gemäß einer weiter bevorzugten Ausführungsform der Erfindung wird mit einem entsprechenden internen oder externen Bewegungsmesssystem (manchmal auch als Bewegungssensor bezeichnet) die Bewegung des Zielvolumens bzw. ein Bewegungssurrogat gemessen. Erfindungsgemäß werden die Messergebnisse mit den mittels des Ionenradiographiedetektors aufgenommenen lonenradiogrammen automatisch, z.B. von einem entsprechend programmierten Mikrocomputer verknüpft und die Bestrahlung wird in Ansprechen auf die verknüpften Daten gesteuert.

Es kann aber auch in einfacher Weise der Wechsel zwischen den Radiographiephasen und den Depositionsphasen in Ansprechen auf die Messergebnisse des Bewegungsmesssystems gesteuert werden.

Z.B. wird als eine Kombination mit dem externen Bewegungssurrogat, die Surrogatinformation verwendet, um eine Bewegungsphase zu bestimmen, in der per Radiogramm verifiziert wird, ob sich das klinische Zielvolumen (CTV) oder einzelne Teststellen des klinischen Zielvolumens (CTV) an der geplanten Position befinden.

Ferner kann das Zielvolumen in der zumindest einen oder der Mehrzahl von Radiographiephasen aus mehr als einer Richtung bestrahlt werden und hiermit wird zumindest ein örtlich mehr als zweidimensionales Ionenradiogramm ("2.5D-Erfassung") aufgenommen. Dies ist für Bestrahlungsplätze vorteilhaft, welche mehr als ein Strahlrohr haben. Hier ist es möglich, Radiogramme aus mehr als einer Richtung zu aufzunehmen und damit eine 2.5D-Erfassung zu ermöglichen. An einer Gantry ist ferner eine "RapicArc" analoge Bestrahlung denkbar, so dass in diesem Fall innerhalb der Bestrahlungsdauer auch hier Radiogramme aus unterschiedlichen Richtungen aufgenommen werden können und durch geeignete Rekonstruktionsalgorithmen somit eine 3D-Bewegung plus Reichweite konstruiert werden kann. Damit ist auch die Aufnahme eine 4DIonenCTs möglich.

Insgesamt liefert dies eine sehr umfassende Information über die Bewegung des Zielvolumens.

Zusammenfassend sollten die erhobenen Daten in Bezug auf die Erwartung in Echtzeit ausgewertet werden, wozu die aus der Fluoroskopie bekannten Methoden verwendbar sind, d.h. unter anderem ein Vergleich zwischen Messung und Digital Rekonstruierter Range Map (DRRM) (auch in 4D, d.h. ein DRRM pro Bewegungsphase des 4DCT) oder es werden Korrelationsmodelle zwischen Radiographie und anderen Surrogaten bzw. parallel aufgenommenen Fluoroskopie/Radiographie-Daten verwendet.

Gemäß einem einfachen Aspekt der Erfindung wird in Ansprechen auf das aufgenommene Ionenradiogramm bei Überschreiten vorbestimmter Grenzwerte, z.B. beim Vergleich zwischen Reichweitensimulation und Energieverlustmessung, ein Interlocksignal generiert, mittels welchem die Bestrahlung unterbrochen wird.

Prinzipiell beschränkt die sich die Erfindung also nicht auf reines Bewegungsmonitoring, sondern es ist auch eine Verifikation der gewonnen DRRMs möglich, so dass z.B. bei zu großen Abweichungen die Bestrahlung unterbrochen und ggf. sogar neu geplant werden kann. Hierbei ist die Erfindung besonders vorteilhaft, da der Ionenradiographiedetektor die Ionenenergie misst und dadurch mit der Teilchenreichweite ein für die Dosis relevanter Faktor direkt erfasst wird (im Gegensatz zu den Verfahren, die lediglich Ersatzgrößen erfassen).

Je nach Kontrast in der bestrahlten anatomischen Region können auch Marker (Goldkugeln, Carbonkugeln, etc.) implantiert werden, um sichtbare Punkte im Ionenradiogramm zu definieren. Die Erfindung ist ferner auch bei inter-fraktionär bewegten oder bei statischen Kopf-Hals Bestrahlungen anwendbar, um z.B. die Lagerung zu ermöglichen oder ein Interlock Signal zur Verfügung zu stellen, falls es unerwartet doch zu Bewegungen kommt.

Erfindungsgemäß wird auch eine Bestrahlungsanlage zur Bestrahlung eines sich bewegenden Zielvolumens mit einem lonenstrahl bereit gestellt, mit welcher das vorstehend beschriebene Verfahren durchgeführt werden kann. Hierzu umfasst die Bestrahlungsanlage:
eine Beschleuniger- und Strahlführungseinrichtung zur Erzeugung und Beschleunigung eines lonenstrahls sowie zum Führen und Richten des lonenstrahls auf das Zielvolumen,
eine Steuereinrichtung zur Steuerung der Bestrahlungsanlage,
eine Einrichtung zum zeitlichen Variieren der Energie des lonenstrahls während der Bestrahlung oder in einer Bestrahlungspause zwischen zumindest einer Radiographiephase und zumindest einer Depositionsphase, insbesondere zusätzlich zu der Reichweitenveränderung für die Dosisdeposition in verschiedenen Isoenergieschichten, mittels welcher Einrichtung
   i) in der zumindest einen Radiographiephase die Energie des lonenstrahls auf eine höhere Radiographienergie eingestellt wird, bei welcher die Reichweite distal des Zielvolumens bzw. distal des Patienten (in Strahlrichtung hinter dem), genauer in dem Ionenradiographiedetektor, liegt, so dass der lonenstrahl das Zielvolumen durchdringt oder durchleuchtet,
   ii) in der zumindest einen Depositionsphase die Energie des lonenstrahls auf eine niedrigere Depositionsenergie eingestellt wird, bei welcher die Reichweite innerhalb des Zielvolumens liegt und der lonenstrahl in dem Zielvolumen abgestoppt wird, um die geplante Dosis in dem Zielvolumen zu deponieren, und
einen distal des Zielvolumens angeordneten Ionenradiographiedetektor zum Aufnehmen von Ionenradiogrammen des Zielvolumens, indem der das Zielvolumen durchdringende lonenstrahl in der Radiographiephase in dem Ionenradiographiedetektor abgestoppt und detektiert wird.

Vorzugsweise schaltet die Einrichtung zum zeitlichen Variieren der Energie des lonenstrahls, insbesondere während der Bestrahlung oder in einer Bestrahlungspause - und zusätzlich zur Reichweitenveränderung zum Anfahren der Isoenergieschichten in der Depositionsphase - in einer zyklischen Abfolge einer Mehrzahl von Radiographiephasen und einer Mehrzahl von Depositionsphasen die Energie des lonenstrahls wechselweise zwischen der Radiographieenergie und der Depositionsenergie hin und her.

Der Ionenradiographiedetektor weist insbesondere eine zeitliche Auflösung auf, die ausreichend ist, um in jeder Radiographiephase ein neues Radiogramm zu erzeugen, um ggf. die Bestrahlung in Echtzeit steuern zu können.

Die Steuereinrichtung steuert die Bestrahlungsanlage vorzugsweise derart, dass in den Depositionsphasen verschiedene Isoenergieschichten des Zielvolumens mit dem lonenstrahl angefahren werden, um jeweils eine vorbestimmte Dosis in den Isoenergieschichten zu deponieren und zumindest vor der Bestrahlung zur Dosisdeposition jeder Isoenergieschicht eine Radiographiemessung mit dem Ionenradiographiedetektor durchgeführt wird.

Vorzugsweise wird ein passiver Energiemodulator verwendet, wobei der lonenstrahl von der Beschleunigereinrichtung zunächst mit der Radiographieenergie erzeugt wird und in der Depositionsphase durch Abbremsung im Material des Energiemodulators auf die Depositionsenergie herabgesetzt wird. Der passive Energiemodulator ist z.B. eine runde Scheibe mit einem tortenstückförmigen Ausschnitt und rotiert im lonenstrahl, so dass der lonenstrahl zyklisch abwechselnd das Material der Scheibe durchdringt und dabei auf die Depositionsenergie abgebremst wird (Depositionsphase) oder ungebremst durch den tortenstückförmigen Ausschnitt passiert (Radiographiephase).

Ferner vorzugsweise steuert die Steuereinrichtung die Bestrahlungsanlage dergestalt, dass die Intensität des lonenstrahls bei der Deposition in dem Zielvolumen höher ist als bei der Radiographiemessung, um die zusätzliche Strahlenbelastung aufgrund der Radiographiemessung für den Patienten niedrig zu halten.

Vorzugsweise unterteilt ein Mikrocomputer in der Vorbereitung der Bestrahlung die Bewegung eines sich zyklisch bewegenden Zielvolumens, z.B. durch Atmung, in mehrere Bewegungsphasen. Wenn ein tortenförmiger Energiemodulator verwendet wird, sind dessen Form und Rotationsgeschwindigkeit vorzugsweise an die Zeitdauer der Bewegungsphasen so angepasst, dass die Zeitdauer der Radiographiephasen kürzer ist als die Zeitdauer der Bewegungsphasen.

Wenn eine Einrichtung zum aktivem Nachführen des lonenstrahls zur Kompensation der Bewegung des Zielvolumens (Beam Tracking) vorhanden ist, steht die Steuereinrichtung vorzugsweise hiermit in Wirkverbindung, um das Beam Tracking in Ansprechen auf die mittels des Ionenradiographiedetektors aufgenommenen Ionenradiogramme zu steuern.

Der Ionenradiographiedetektor ist ein energie- und ortsauflösender Detektor der jeweils ein energie- und lateral zweidimensional ortsaufgelöstes Ionenradiogramm zumindest von Teilen des internen Zielvolumens (ITV) aufnimmt. Eine Recheneinrichtung ermittelt dann die Reichweite des lonenstrahls nach dem Durchdringen des Zielvolumens für jeden der Rasterpunkte und erstellt eine zweidimensionale Karte der Reichweite des lonenstrahls nach dem Durchdringen des Zielvolumens.

Die vorzugsweise umfasste Scann-Einrichtung zum Scannen des lonen-Bleistiftstrahls (Durchmesser typischerweise einige Millimeter) wird von der Steuereinrichtung so gesteuert, dass der lonenstrahl zur Dosisdeposition zwei- oder dreidimensional über das Zielvolumen gescannt wird und zur Radiographie zumindest über einen Teil der lateralen Fläche des Zielvolumens unabhängig von dem Scannen zur Deposition, weil zeitlich konsekutiv, gewobbelt wird. Zum Scannen und Wobbeln können dieselben Scannmagnete verwendet werden, es ist aber denkbar separate Magnete für das Wobbeln einzubauen, z.B. wenn die Scannmagnete für das Wobbeln nicht schnell genug sind.

Die Steuereinrichtung steuert die Scanneinrichtung (mit oder ohne separate Wobbling-Magnete) z.B. so, dass der lonenstrahl bei der Dosisdeposition über das klinische Zielvolumen (CTV, ICRU 50) gescannt wird und beim Radiographieren über eine größere laterale Fläche als das klinische Zielvolumen, insbesondere über das interne Zielvolumen (ITV, ICRU 62) gewobbelt wird.

Die vorstehend beschriebene Reichweiten-Simulationsrechnung wird typischerweise von einem entsprechend programmierten Mikrocomputer durchgeführt, welcher auch den Vergleich mit den tatsächlich ermittelten Reichweiten durchführt und anschließend, vorzugsweise in Echtzeit, Steuersignale erzeugt, mit denen z.B. die Bestrahlung adaptiert oder unterbrochen wird. Gleiches gilt für die Ausführungsform bei welcher die mehrdimensionalen tatsächlich gemessenen Radiogramme mit der simulierten Sollwert-Karte verglichen werden.

Insbesondere nimmt die Steuereinrichtung die Messergebnisse des Bewegungsmesssystems und die Ionenradiogramme des Ionenradiographiedetektors auf, verknüpft automatisch die Messergebnisse und die Ionenradiogramme und steuert anschließend die Bestrahlung in Ansprechen auf diese verknüpften Daten.

Alternativ steuert die Steuereinrichtung den Wechsel zwischen den Radiographiephasen und den Depositionsphasen in Ansprechen auf die Messergebnisse des Bewegungsmesssystems. Weiter vorzugsweise weist die Bestrahlungsanlage mehrere Strahlrohre und/oder eine drehbare Gantry auf, womit das Zielvolumen aus mehr als einer Richtung bestrahlt werden kann, um ein örtlich mehr als zweidimensionales Ionenradiogramm aufzunehmen.

Das beschriebene Verfahren und die Bestrahlungsanlage sind insbesondere ausgebildet für die Tumortherapie. Sie können aber auch eingesetzt werden, um ein Zielvolumen zu bestrahlen, welches nicht einem menschlichen oder tierischen Körper angehört. Z.B. kann ein Phantom, insbesondere mit einem Zielvolumen zur Bewegungssimulation bestrahlt werden.

Im Folgenden wird die Erfindung anhand von Ausführungsbeispielen und unter Bezugnahme auf die Figuren näher erläutert, wobei gleiche und ähnliche Elemente teilweise mit gleichen Bezugszeichen versehen sind und die Merkmale der verschiedenen Ausführungsbeispiele miteinander kombiniert werden können.

### Kurzbeschreibung der Figuren

Es zeigen:
- Fig. 1: eine schematische Darstellung zur Bestrahlung eines bewegten Zielvolumens mit einem aufgestreuten lonenstrahl
- Fig. 2: eine schematische Darstellung zur Bestrahlung eines bewegten Zielvolumens mit einem gescannten lonenstrahl
- Fig. 3: eine schematische Darstellung einer Bestrahlungsanlage mit einem aufgestreuten lonenstrahl gemäß Stand der Technik
- Fig. 4: eine schematische Darstellung einer Bestrahlungsanlage mit einem gescannten lonenstrahl gemäß Stand der Technik
- Fig. 5: eine schematische Darstellung einer Bestrahlungsanlage mit einem gescannten lonenstrahl und einem Energiemodulator gemäß einer Ausführungsform der Erfindung
- Fig. 6: eine schematische Darstellung einer Bestrahlungsanlage mit gescanntem lonenstrahl und einem Energiemodulator gemäß einer weiteren Ausführungsform der Erfindung
- Fig. 7: eine schematische Darstellung einer Bestrahlungsanlage mit einem nachgeschalteten Linearbeschleuniger gemäß einer weiteren Ausführungsform der Erfindung
- Fig. 8: eine schematische Darstellung der Bestrahlung eines Zielvolumens kombiniert mit einem Bewegungsmesssystem
- Fig. 9: eine schematische Darstellung einer Bestrahlung eines Zielvolumens aus zwei unterschiedlichen Richtungen
- Fig. 10: eine schematische Darstellung einer Bestrahlung eines Zielvolumens mit rotierender Gantry
- Fig. 11: eine Darstellung des Zeitablaufs der Radiographiephasen und Depositionsphasen gemäß einer Ausführungsform der Erfindung
- Fig. 12: eine Darstellung der Radiographiephasen und Depositionsphasen gemäß einer weiteren Ausführungsform der Erfindung
- Fig. 13: eine Darstellung der Radiographiephasen und Depositionsphasen gemäß einer weiteren Ausführungsform der Erfindung
- Fig. 14: ein Ablaufschema einer Bestrahlung gemäß einer Ausführungsform der Erfindung
- Fig. 15: ein Ablaufschema einer Echtzeitauswertung mit DRRM gemäß einer Ausführungsform der Erfindung
- Fig. 16: ein Ablaufschema einer Kombination der Bestrahlung mit Surrogatmessung gemäß einer Ausführungsform der Erfindung

### Detaillierte Beschreibung der Erfindung

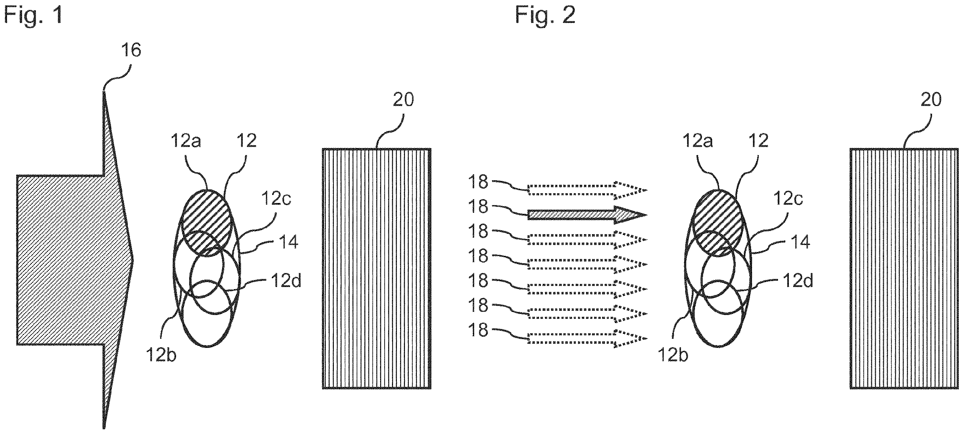

Fig. 1 zeigt einen Tumor als klinisches Zielvolumen 12 (CTV) gemäß ICRU 50 in einer Referenzbewegungsphase 12a. Der Tumor 12 bewegt sich innerhalb einer Einhüllenden innerhalb derer beispielhaft die Bewegungszustände 12a, 12b, 12c und 12d abgebildet werden. Die Einhüllende bildet das interne Zielvolumen 14 (internal target volume, ITV) gemäß ICRU 62.

Um mittels der Radiographiemessung mit dem Ionenradiographiedetektor 20 Informationen über Reichweitenverteilung im gesamten internen Zielvolumen 14 zu erhalten, sind bei einer passiven Bestrahlung mit aufgestreutem lonenstrahl 16 in der Regel keine weiteren Maßnahmen erforderlich, da ohnehin das gesamte interne Zielvolumen 14 gleichzeitig bestrahlt wird.

Fig. 2 zeigt die Bestrahlung des klinischen Zielvolumens 12 mit einem gescannten lonenstrahl 18. Um mittels der Radiographiemessung mit dem Ionenradiographiedetektor 20 Informationen über Reichweitenverteilung im gesamten internen Zielvolumen 14 zu erhalten, wird der lonenstrahl hierbei über das gesamte interne Zielvolumen 14 gewobbelt, was schematisch durch die gepunkteten Pfeile 18 symbolisiert ist. Beim Wobbeln wird der feine Bleistiftstrahl schnell über die laterale Ausdehnung des gesamten internen Zielvolumens 14 gefahren, wovon in Fig. 2 nur eine Dimension dargestellt ist, da die zweite laterale Dimension senkrecht zur Zeichenebene liegt.

Gegebenenfalls wird die gemessene Reichweitenverteilung z.B. mit einer Digital Rekonstruierten Range Map (DRRM) verglichen. Alternativ kann aber auch durch Vergleich der mittels des Ionenradiographiedetektors 20 durchgeführten Radiographiemessung an der aktuellen Rasterposition oder an mehreren repräsentativ angefahrenen Rasterpositionen mit einer DRRM Information über die aktuelle Strahlposition bzw. verschiedenen Strahlpositionen gewonnen werden.

Mit anderen Worten ist in den Digital Rekonstruierten Range Maps (DRRM) der erwartete Reichweiteverlust des Radiographiestrahls, der aus seiner bestimmten Richtung an einer bestimmten Position mit einer bestimmten Energie durch ein CT (oder eine Phase eines 4D-CTs) gestrahlt wird, für eine Vielzahl von Strahlen gespeichert. Während der Radiographie werden die Messwerte der Radiographiemessung mit der DRRM verglichen. Hiermit wird z.B. die Bewegungsphasenbestimmung eines Bewegungssurrogats verifiziert oder es wird eine unabhängige Festlegung der Bewegungsphase in Ansprechen auf die Radiographiemessung vorgenommen.

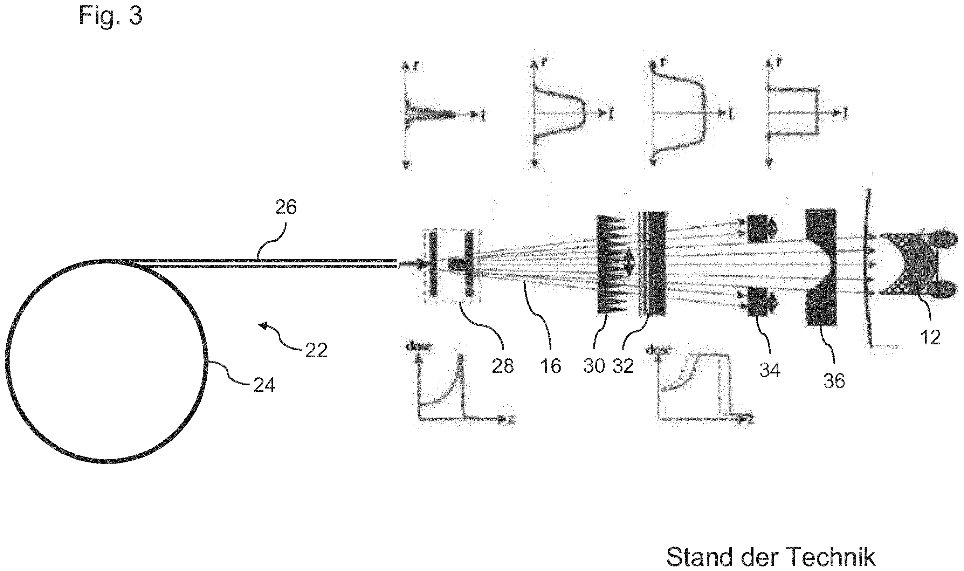

Fig. 3 zeigt eine bekannte Bestrahlungsanlage 1 mit einer Beschleunigereinrichtung 22 umfassend ein Zyklotron 24 und eine Strahlführungseinrichtung 26. Der lonenstrahl 16 wird mit einem Streusystem 28 (scattering system) aufgestreut. Anschließend wird die Reichweite mit einem Reichweitenmodulator 30 (range modulator)und einem Reichweitenschieber 32 (range shifter) verbreitert. Nachfolgend wird der aufgestreute lonenstrahl 16 mit einem Kollimator 34 auf die Ausdehnung des internen Zielvolumens kollimiert. Das Dosisfeld wird mittels eines Kompensators 36 an die distale Kontur des Zielvolumens angepasst. Der ursprünglich feine lonenstrahl 16, so wie er aus der Beschleunigereinrichtung 22 emittiert wird, wird durch das Streusystem sowie diverse sich anschließende passive Strahlformungseinrichtungen an das Zielvolumen angepasst. Es handelt sich hierbei um ein vollständig passives Strahlmodellierungssystem.

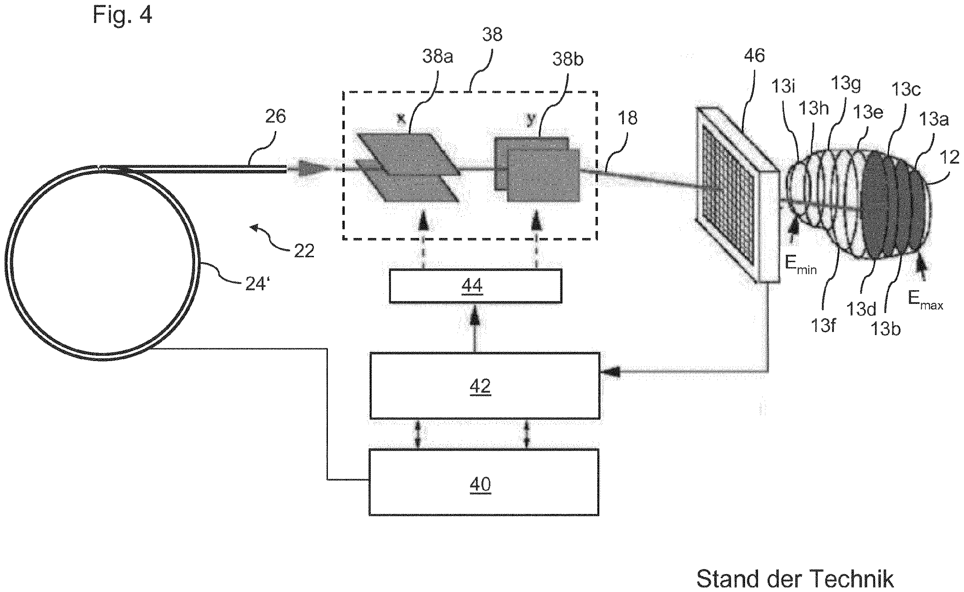

Fig. 4 zeigt eine bekannte Bestrahlungsanlage 1 mit einem intensitätsgesteuerten magnetischen Scannsystem, so wie es z.B. bei der GSI in Darmstadt eingesetzt wird. Die Beschleunigereinrichtung 22 umfasst in diesem Beispiel ein Synchrotron 24' sowie eine Strahlführungseinrichtung 26, welche den lonenstrahl in die Bestrahlungskammer (nicht dargestellt) führt, um dort das Zielvolumen 12 zu bestrahlen. Der feine lonenstrahl 18, auch als Pencil Beam (deutsch: Bleistiftstrahl) wird von einer Scanneinrichtung 38, welche schnelle Scannmagnete 38a, 38b zum Scannen des lonenstrahls 18 in X- und Y-Richtung umfasst, über die laterale Ausdehnung des Zielvolumens gescannt. Um das Zielvolumen dreidimensional abzuscannen wird der Bragg-Peak über eine Mehrzahl von Isoenergieschichten 13a bis 13i gescannt. Der lonenstrahl 18 ist z.B. ein 80 bis 430 MeV/u ¹²C-Ionenstrahl. Die entsprechenden Strahlparameter des lonenstrahls 18, werden vom Synchrotronkontrollsystem 40 gesteuert und gepulst von dem Synchrotron 24' bereit gestellt. Typischerweise werden diese Isoenergieschichten 13a bis 13i von distal (höchste Energie, Eₘₐₓ) nach proximal (niedrigste Energie, Eₘᵢₙ) abgescannt. Das Abscannen des Zielvolumens 12 wird zunächst in einer Vorbereitungsphase, der sogenannten Bestrahlungsplanung, vorbereitet, in welcher ein Bestrahlungsplan berechnet und festgelegt wird, welcher im Therapiekontrollsystem 42 hinterlegt ist. Das Therapiekontrollsystem 42 steht in wechselweiser Wirkverbindung mit dem Synchrotronkontrollsystem 40 und steuert unter anderem die Leistungsversorgung 44 der Scanneinrichtung 38, um den jeweiligen Rasterpunkt für die Dosisdeposition anzusteuern. Ferner wird die Strahlposition mit einem Strahlpositionsmonitor 46 überwacht und an das Therapiekontrollsystem 42 übermittelt.

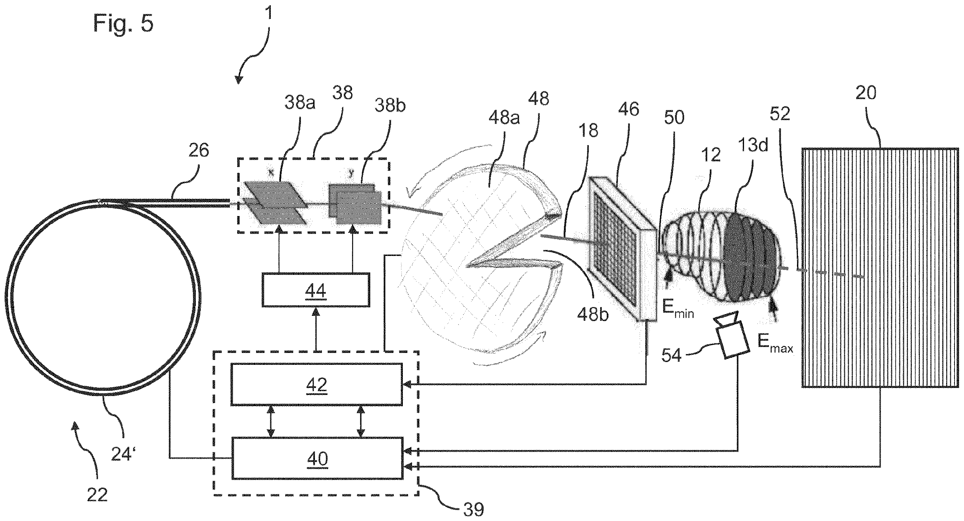

Bezug nehmend auf Fig. 5 ist eine Bestrahlungsanlage mit Scannsystem 38 entsprechend Fig. 4 dargestellt, wobei nun erfindungsgemäß ein tortenförmiger Energiemodulator 48 proximal des Zielvolumens bzw. proximal des Strahlpositionsmonitors 46 angeordnet ist. In diesem Beispiel ist der Energiemodulator 48 distal der Scanneinrichtung 38 angeordnet. Die eigentliche Bestrahlung des Zielvolumens 12, d.h. die Dosisdeposition wird mit einer Therapieenergie bzw. Depositionsenergie E durchgeführt. Die Beschleunigereinrichtung 22 liefert allerdings die erheblich höhere Radiographieenergie E'= E + dE, wobei dE dem Energieverlust beim Durchstrahlen des tortenförmigen Energiemodulators 48 entspricht. Der Energiemodulator 48 rotiert, um die zeitliche Abfolge zwischen Depositionsphasen und Radiographiephasen zu definieren. In den Zeiten, in denen der Energiemodulator durchstrahlt wird, nämlich in dem massiven Bereich 48a in dem sich das Modulationsmaterial befindet, moduliert der Energiemodulator 48 die Ionenstrahlenergie von der Radiographieenergie E' auf die Depositionsenergie E herunter, so dass das Zielvolumen wie üblich bestrahlt werden kann, was durch die durchgezogene Linie 50 repräsentiert ist. In dem Zeitintervall, in dem der lonenstrahl die Aussparung 48b des Energiemodulators passiert, tritt kein Energieverlust dE auf, so dass am Patienten die Radiographieenergie E'= E + dE ankommt. Die Radiographieenergie E' bzw. der Energieverlust dE wird ausreichend groß gewählt, so dass das Zielvolumen und im Fall einer therapeutischen Bestrahlung, der gesamte Patient von dem lonenstrahl vollständig durchdrungen wird, was durch die gestrichelte Linie 52 repräsentiert ist. In diesem Fall kann mit dem orts- und energieauflösenden lonenradiographiedetektor 20, welcher distal des Zielvolumens 12 angeordnet ist und eine Größe besitzt, welche zumindest das gesamte interne Zielvolumen abdecken sollte, ortsaufgelöst der Energieverlust im Zielvolumen 12 bzw. im Patienten gemessen werden. Folglich wird in dieser Radiographiephase mit der Radiographieenergie E' eine Ionen-Radiographiemessung mit dem Ionenradiographiedetektor 20 durchgeführt. Beispielsweise beträgt die Radiographieenergie etwa E' = 600 MeV/u, die Depositionsenergie etwa E = 250 MeV/u und der Energieverlust in dem Energiemodulator etwa dE = 350 MeV/u.

Der orts- und energieauflösende Ionenradiographiedetektor 20 in diesem Beispiel umfasst einen Stapel von bis zu 61 parallelen Ionisationskammern zwischen denen jeweils Absorberplatten aus PMMA eingesetzt sind. Die Dicke der Absorberplatten wird je nach Anforderung zwischen 0,5 mm und 5 mm gewählt. Der Ionenradiographiedetektor 20 kann ferner einen festen oder variablen Vorabsorber umfassen, welcher die Wasseräquivalent-Reichweite um 90 mm bzw. bis zu 90 mm herabsetzt. Die aktive Fläche des Ionenradiographiedetektors 20 beträgt beispielsweise 300 x 300 mm², um zumindest ein Messfeld von 200 x 200 mm² für den lonenstrahl 18 bereit zu stellen.

Der Energiemodulator 48 steht in Wirkverbindung mit der Steuereinrichtung 39 der Bestrahlungsanlage 1. Die Steuereinrichtung 39 kann die Rotation des Energiemodulators 48 steuern und/oder der Energiemodulator 48 gibt eine Rückmeldung über seine jeweilige Winkelposition an die Steuereinrichtung 39, unter anderem um die Steuerung der Beschleunigereinrichtung 22, der Scanneinrichtung 38 und die mittels des rotierenden Energiemodulators 48 definierten Radiographiephasen und Depositionsphasen zeitlich aufeinander abzustimmen bzw. zu synchronisieren.

Die Bestrahlungsanlage 1 umfasst zusätzlich noch ein an sich bekanntes Bewegungsmesssystem 54, welches eine Information über die Bewegung des Zielvolumens 12 an die Steuereinrichtung 39 übermittelt. Dies kann eine direkte Bewegungsinformation mit einem internen Bewegungsmesssystem oder eine Surrogatinformation mit einem externen Bewegungsmesssystem sein.

In Bezug auf die Verwendung des zusätzlichen internen oder externen Bewegungsmesssystems 54 kommen z.B. folgende Systeme in Betracht. Als internes Bewegungsmesssystem:
- kV/MV Imaging (Photonen) Fluoroskopie
   ∘ mit implantierten Markern
   ∘ ohne implantierte Marker
- implantierte elektromagnetische Transponder
- Ultraschall
und als externes Bewegungsmesssystems zur Messung eines Bewegungssurrogats:
- Messung des Patientenatems:
   ∘ Volumenmessung
   ∘ Temperaturmessung
   ∘ Messung der Luftflussgeschwindigkeit
- Messung Patientenoberfläche
   ∘ Filmen aufgebrachte Marker (z.B. Infrarotemitter)
   ∘ Stereokamera zum Filmen der Körperoberfläche

Es können auch verschiedene Bewegungsmesssysteme kombiniert werden.

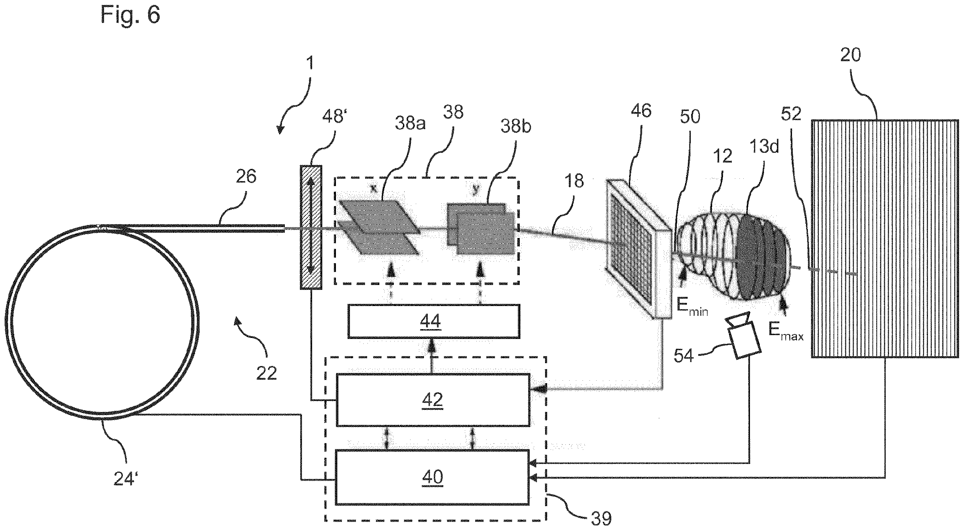

Bezug nehmend auf Fig. 6 ist eine alternative Ausführungsform der Erfindung dargestellt, bei welcher der Energiemodulator proximal der Scanneinrichtung 38 angeordnet ist. In diesem Beispiel ist der Energiemodulator als binäre Energiemodulatorplatte 48' ausgebildet, welche die Reduktion der Strahlenergie von der Radiographieenergie auf die Depositionsenergie durch Herein- und Herausfahren der Energiemodulatorplatte 48' in bzw. aus dem Strahlengang des lonenstrahls 18 definiert. Durch das Herausfahren der Energiemodulatorplatte 48' wird die Strahlenergie von der Depositionsenergie E auf die Radiographieenergie E'= E + dE erhöht, so dass das Zielvolumen 12 bzw. bei der Tumortherapie der gesamte Patient vom lonenstrahl durchdrungen wird, um die Radiographiemessung durchführen zu können. Durch Hereinfahren der Energiemodulatorplatte 48' wird die von der Beschleunigereinrichtung gelieferte Radiographieenergie E' um dE auf die Depositionsenergie E herabgesetzt, um das Zielvolumen 12 so zu bestrahlen, dass die in dem Bestrahlungsplan festgelegte Dosis deponiert wird, was in Fig. 6 durch die durchgezogene Linie 50 repräsentiert ist, wobei hier beispielhaft die von distal gesehen vierte Isoenergieschicht 13d bestrahlt wird.

Da in diesem Beispiel der Energiemodulator 48' proximal der Scanneinrichtung 38 angeordnet ist, müssen die Scanneinrichtung 38 und ggf. noch weitere distal des Energiemodulators 48' angeordnete Magnete der Strahlführung nachjustiert werden. Dies kann in Echtzeit mit entsprechend schnellen Magnetsystemen, gesteuert durch die Steuereinrichtung 39, realisiert werden. Die Positionierung des Energiemodulators vor der Scanneinrichtung 38 hat den Vorteil einer niedrigeren Patientenbelastung durch Fragmente, welche im Energiemodulator erzeugt werden. Die Positionierung des Energiemodulators distal der Scanneinrichtung bzw. vor dem Patienten, wie in Fig. 5 dargestellt, hat den Vorteil, dass auf ein entsprechendes Nachregeln der Scanneinrichtung 38 und weiterer Strahlführungselemente verzichtet werden kann.

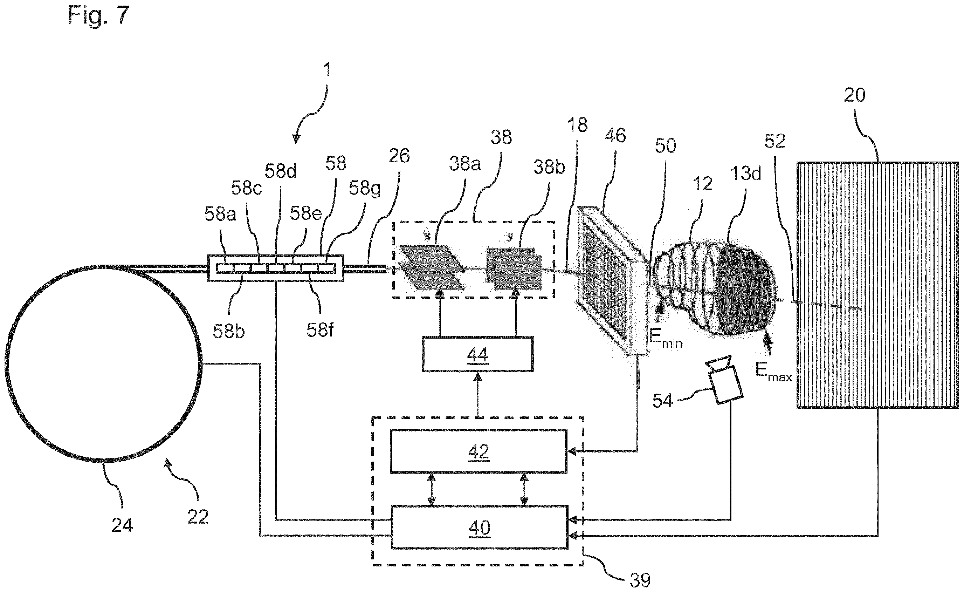

Fig. 7 zeigt eine weitere Ausführungsform, bei welcher die Energievariation zwischen der Radiographieenergie und der Depositionsenergie von der Beschleunigereinrichtung 22 durchgeführt wird. Hierzu eignet sich z.B. ein Zyklotron 24 mit nachgeschaltetem Linearbeschleuniger 58. Im Übrigen entspricht der Aufbau im Wesentlichen demjenigen in Fig. 5 und 6. Im Unterschied zu den in Fig. 5 und 6 verwendeten passiven Energiemodulatoren 48, 48' kann die Energievariation zwischen der Depositionsenergie und der Radiographieenergie in diesem Beispiel mit dem nachgeschalteten Linearbeschleuniger 58 bewerkstelligt werden. Für die Depositionsphase werden eine oder mehrere der Beschleunigerkavitäten 58a bis 58g abgeschaltet, um die Energie des lonenstrahls von der Radiographieenergie auf die Depositionsenergie herabzusetzen. Hierzu kann z.B. der Cyc-LINAC verwendet werden, welcher in dem vorstehend zitierten Review Artikel von Ugo Amaldi et al beschrieben ist.

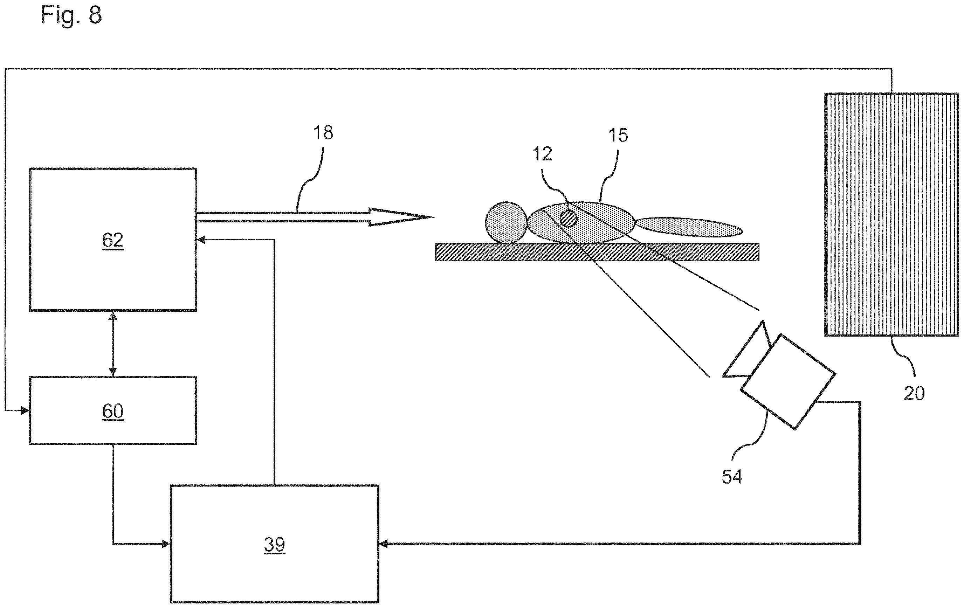

Fig. 8 zeigt die Kombination des Bewegungsmesssystems 54 mit der Radiographiemessung mittels des Radiographiedetektors 20 gemäß der vorliegenden Erfindung. Das an sich bekannte Bewegungsmesssystem 54 übermittelt die Daten über die Bewegung des Zielvolumens an die Steuereinrichtung 39. Der Ionenradiographiedetektor 20 nimmt in der Radiographiephase mittels des das Zielvolumen 12 bzw. den Patienten 15 durchstrahlenden lonenstrahls 18 Ionenradiogramme des Zielvolumens (und des umgebenden Gewebes) auf. Die Steuereinrichtung 39 vergleicht die Ionenradiographiemessungen 60 mit den Messungen des Bewegungsmesssystems 54, welches z.B. ein Surrogat in Form eines Stereokamerabildes der Patientenbrust sein kann. Mit dem Bewegungsmesssystem 54 kann zunächst in an sich bekannter Weise die Bewegungsphase des Patienten bestimmt werden. Wenn die Steuereinrichtung 39 feststellt, dass die mittels des Bewegungsmesssystems 54 bestimmte Bewegungsphase - welche in die Strahlapplikation 62, z.B. das Gating oder Beam Tracking einfließt - mit den in dieser Bewegungsphase erwarteten Ionenradiographieergebnissen übereinstimmt, wird die Bestrahlung fortgesetzt. Wenn andererseits das lonenradiographieergebnis vorbestimmte Schwellwerte der Übereinstimmung mit den erwarteten Ionenradiographieergebnissen übersteigt, bricht die Steuereinrichtung 39 die Strahlapplikation 62 ab oder adaptiert diese.

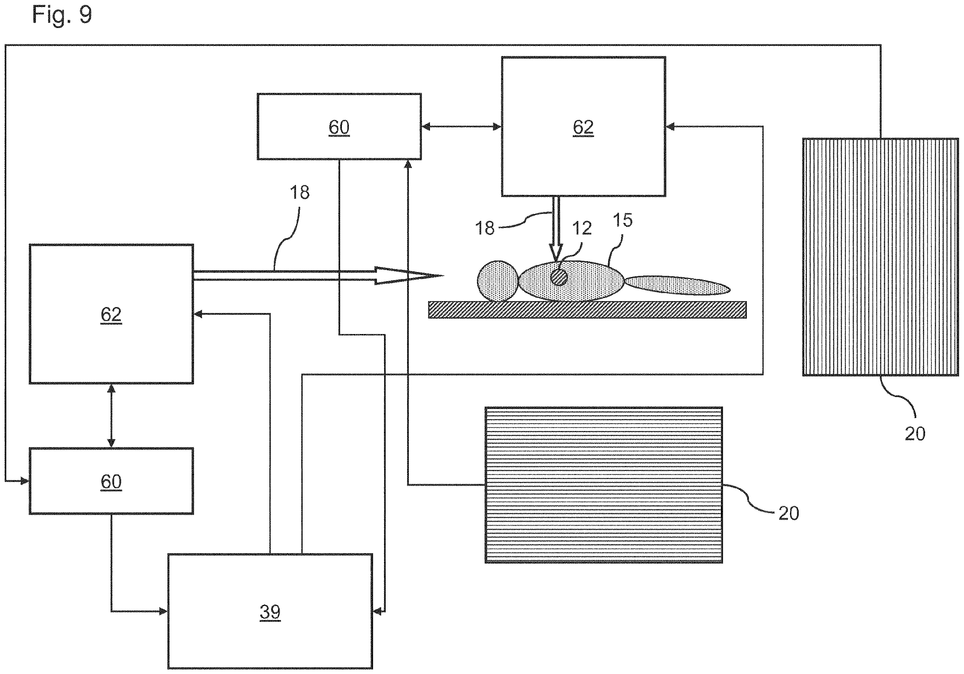

Fig. 9 zeigt eine Ausführungsform der Erfindung, bei welcher das Zielvolumen 12 bzw. der Patient 15 aus zwei unterschiedlichen Richtungen mit dem lonenstrahl 18 bestrahlt wird. Zur Aufnahme der Ionenradiogramme sind entsprechend zwei Ionenradiographiedetektoren 20 vorgesehen. Wenn wie in dem vorliegenden Beispiel aus zumindest zwei verschiedenen Richtungen mit dem lonenstrahl 18 bestrahlt wird, stehen Ionenradiogramme aus verschiedenen Richtungen zur Verfügung. Die mit den Ionenradiographiemessungen 60 aus zumindest zwei verschiedenen Richtungen gewonnene Information ist einer rein zweidimensionalen Information eines einzelnen Radiogramms überlegen, stellt jedoch noch keine volle dreidimensionale Radiographieinformation dar. Daher wird diese Art der Bestrahlung als 2.5D-Radiographie bezeichnet. In jedem Fall kann die Strahlapplikation 62 selektiv mit dem jeweiligen Ionenradiogramm gesteuert werden.

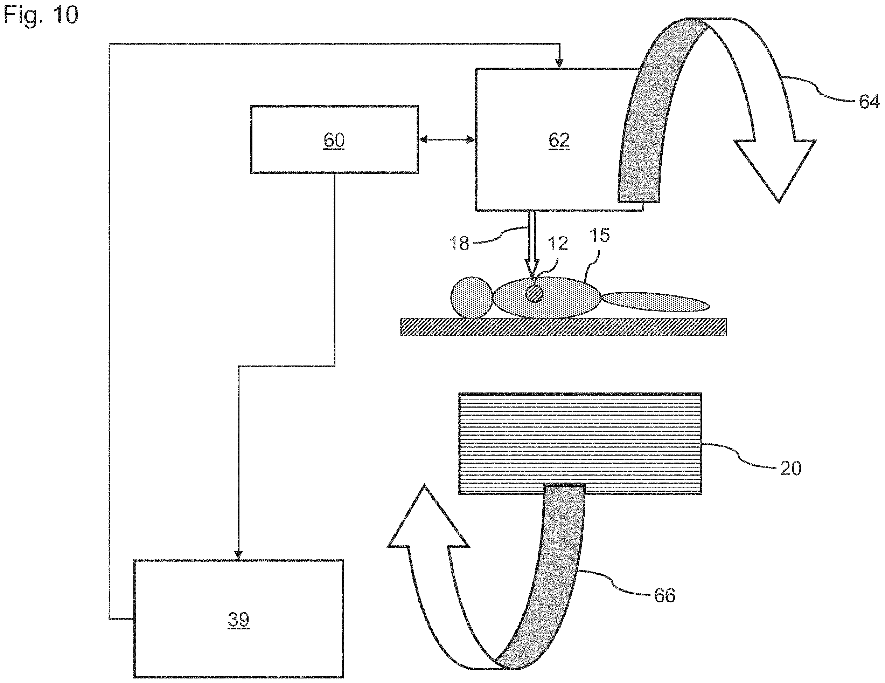

Fig. 10 zeigt ein Ausführungsbeispiel mit einer rotierenden Gantry, wobei die Rotation der Strahlapplikation 62 mit dem Pfeil 64 symbolisiert ist. Der Ionenradiographiedetektor 20 wird entgegengesetzt der Strahlapplikation ebenfalls mit der Gantry (nicht dargestellt) mitrotiert, was mit dem Pfeil 66 symbolisiert ist. Hiermit können folglich ähnlich dem sogenannten RapidArc-Verfahren (Photonen) der Strahlaustritt und der Ionenradiographiedetektor 20 während der Radiographiebestrahlung um den Patienten gedreht werden, so dass 3D-Ionenradiogramme ähnlich wie bei einem Röntgen-CT erstellt werden können, allerdings hier zusätzlich mit der dem Ionenradiogramm inhärenten Reichweiteninformation.

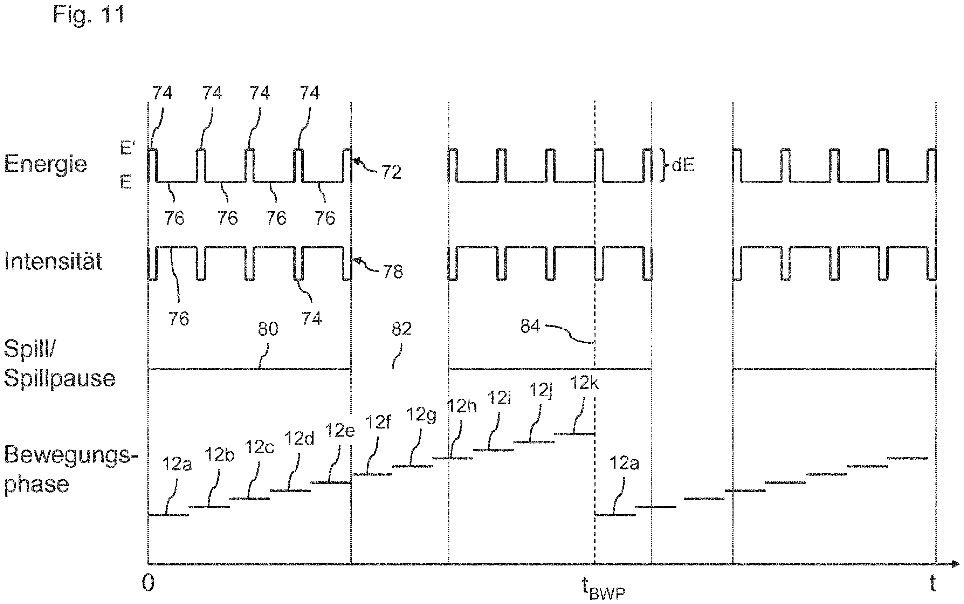

Fig. 11 zeigt ein Ausführungsbeispiel für eine zeitliche Abfolge der Radiographiephasen und Depositionsphasen. Der oberste Graph 72 zeigt die Energie des lonenstrahls, welche zwischen Radiographiephasen 74 mit höherer Radiographieenergie und Depositionsphasen 76 mit niedrigerer Depositionsenergie zyklisch hin- und hergeschaltet wird. Gegenläufig zur Energie 72 des lonenstrahls wird die Intensität 78 des lonenstrahls gesteuert, d.h. dass in den Radiographiephasen 74 eine geringere Intensität des lonenstrahls appliziert wird als in den Depositionsphasen 76. Daher ist die Strahlenbelastung bei dem erfindungsgemäßen Verfahren vorteilhafterweise relativ niedrig. Der Faktor um den die Ionenstrahlintensität in der Radiographiephase gegenüber der Depositionsphase reduziert werden kann, hängt z.B. von der Geschwindigkeit der Wobbling-Magnete und der Charakteristik des Radiographiedetektors 20 ab. Je langsamer die Wobbling-Magnete und der Ionenradiographiedetektor 20 arbeiten, desto größer kann in der Regel die Intensitätsreduktion gewählt werden. Grundsätzlich erscheint eine Reduktion der Intensität in der Radiographiephase gegenüber der Depositionsphase um Faktor 10 bis 100 möglich.

In dem in Fig. 11 dargestellten Beispiel ist der Zyklus des Wechsels zwischen den Radiographiephasen und den Depositionsphasen auf das Scannen des Zielvolumens 12 mit der Scanneinrichtung 38 zur Deposition abgestimmt. Zum Beispiel erfolgt der Wechsel von der Depositionsphase 76 in die Radiographiephase 74 nach einer vorgegebenen Anzahl von bestrahlten Rasterpunkten, z.B. nach 100 Rasterpunkten. Somit entspricht die Zeitdauer der Depositionsphase 76 der Zeitdauer der Bestrahlung von 100 Rasterpunkten. Die Zeitdauer der Radiographiephasen 74 wird deutlich kürzer gewählt, als die Zeitdauer der Depositionsphasen 76.

Das gezeigte Beispiel bezieht sich auf eine Bestrahlungsanlage 1 mit einem Synchrotron 24'. Wie der Fachmann weiß, wird der lonenstrahl aus einem Synchrotron in sogenannten Spills diskontinuierlich extrahiert, wobei die Spills mit 80 und die Spillpausen mit 82 bezeichnet sind. Die Bewegung des Zielvolumens 12 ist in elf Bewegungsphasen 12a bis 12k unterteilt, welche in diesem Beispiel in zwei Spills hineinragt. Der Bewegungszyklus 12a bis 12k endet in diesem Beispiel bei t_{BWP} an der gestrichelten Linie 84, an welcher ein neuer Bewegungszyklus mit der ersten Bewegungsphase 12a beginnt, usw. Soweit sich die Bewegung des Zielvolumens 12 auf die Atmung eines Patienten bezieht, dauert ein Bewegungszyklus (Atemzyklus) typischerweise im Bereich von 5 Sekunden.

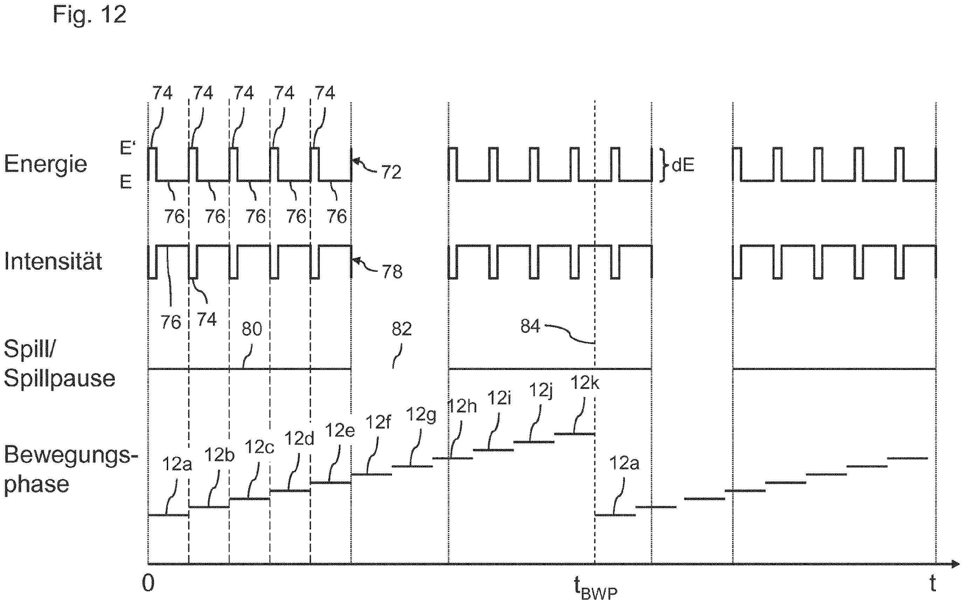

Fig. 12 zeigt einen alternativen Zyklus an Radiographiephasen 74 und Depositionsphasen 76, wobei der Radiographie-Depositions-Zyklus an die Bewegungsphasen 12a bis 12k angepasst ist, bzw. mit den Bewegungsphasen synchronisiert ist. Der Wechsel von einer Depositionsphase 76 in eine Radiographiephase 74 erfolgt zeitgleich mit dem Wechsel von einer Bewegungsphase zur nächsten. Hierzu wird der Zyklus aus Radiographiephasen und Depositionsphasen durch die Messung des Bewegungsmesssystems 54 getriggert, dahingehend, dass der Wechsel von der Depositionsphase 76 zur Radiographiephase 74 erfolgt, wenn das Bewegungsmesssystem 54 den Wechsel zur nächsten Bewegungsphase detektiert. Dies hat den Vorteil, dass für jede Bewegungsphase eine separate Radiographiemessung durchgeführt wird. In diesem Beispiel sind demnach der Zyklus der Radiographiephasen und Depositionsphasen mit dem Zyklus der Bewegungsphasen synchronisiert. Bezug nehmend auf Fig. 5 bis 7 wird die Synchronisierung mittels der Steuereinrichtung 39 vorgenommen.

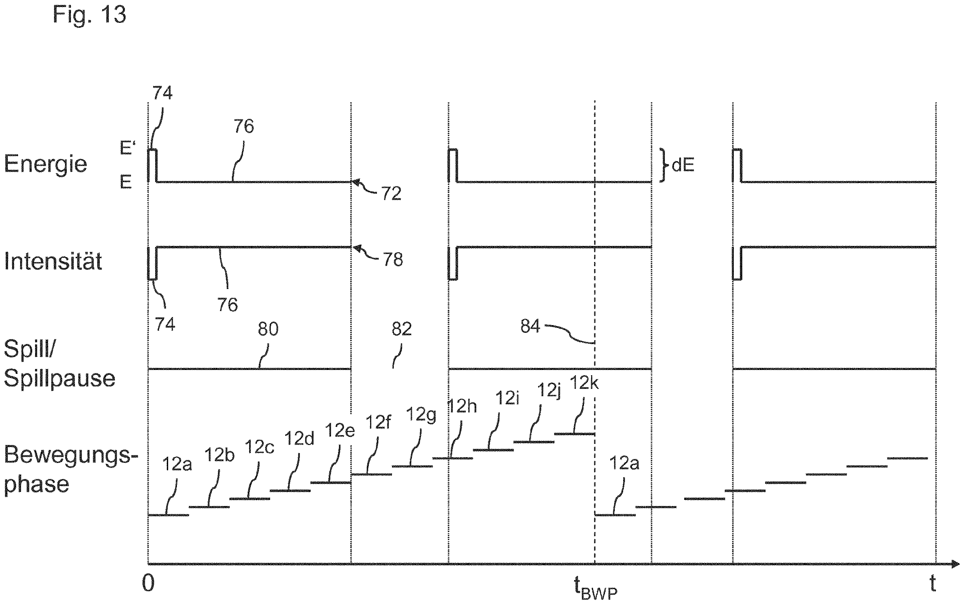

Bezug nehmend auf Fig. 13 kann der Zyklus aus Radiographiephasen und Depositionsphasen auch an das Intervall der Strahlextraktion, am Beispiel des Synchrotrons 24' mit der Zeitdauer des Spills 80 synchronisiert sein. Vorliegend bedeutet das, dass zu Beginn jedes Spills 80 eine Radiographiephase vorliegt und für den Rest des Spills Dosis im Zielvolumen 12 deponiert wird. Auch diese Synchronisierung kann mit einer entsprechend programmierten Steuereinrichtung 39 durchgeführt werden.

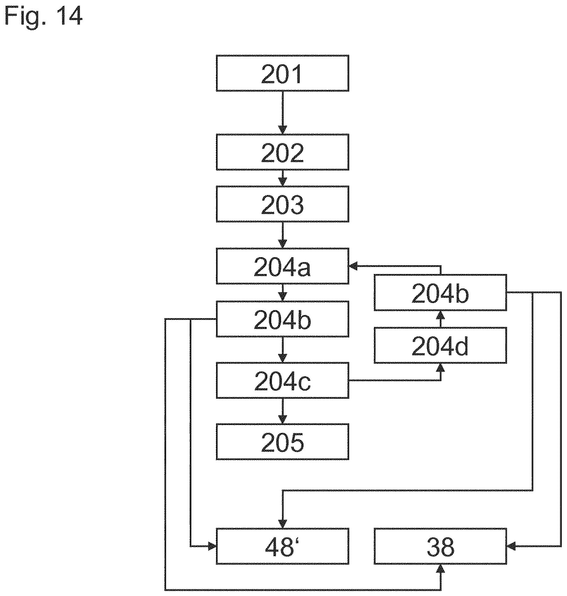

Fig. 14 zeigt ein Ablaufdiagramm zur Bestrahlung im Rahmen der vorliegenden Erfindung mit Radiographiephasen und (optionaler) Echtzeitintensitätsregelung sowie (optionalem) Wobbeln mit dem Scannsystem 38.

In Schritt 201 wird die Strahlentherapie vorbereitet, was Diagnose, Bildgebung und 4D-Bestrahlungsplanung etc. umfasst und dem Fachmann grundsätzlich bekannt ist.

In Schritt 202 werden die Radiographieparameter festgelegt, umfassend die Radiographieenergie E', die Strahlintensität 78 in den Radiographiephasen 74 und/oder die Feldgröße für die Radiographiemessung und/oder Häufigkeit sowie Zeitpunkt bzw. Zyklus der Radiographiemessungen.

In Schritt 203 wird ein DRRM berechnet, z.B. durch ortsaufgelöste Berechnung des Energieverlustes des lonenstrahls im Patientenkörper 15 auf Basis der CT- und Bestrahlungsplanungsdaten (Strahlrichtung, Energie etc.).

Schritt 204a definiert eine Depositionsphase 76 mit der geringeren Depositionsenergie E und der höheren Strahlintensität 78, im Falle einer Tumortherapie also eine Phase therapeutischer Bestrahlung. Wie bereits vorstehend beschrieben, kann eine Depositionsphase 76 die Bestrahlung einer Vielzahl von Rasterpunkten umfassen.

In Schritt 204b werden die Parameter der Bestrahlungsanlage 1 für die nachfolgende Radiographiemessung angepasst. Dies umfasst insbesondere die Variierung der Strahlenergie 72 von der Depositionsenergie E auf die Radiographieenergie E' z.B. mit dem Energiemodulator 48, 48' sowie ggf. die Reduktion der Strahlintensität 78. Beides kann in Echtzeit vorgenommen werden.

In Schritt 204c wird die Radiographiemessung durchgeführt, z.B. indem das gesamte interne Zielvolumen 14 (ITV) durch Wobbeln mit dem lonenstrahl 18 überstrichen wird. Hierzu werden beispielsweise die Scannermagnete 38a, 38b von der Steuereinrichtung 39 entsprechend angesteuert. Abhängig vom Ergebnis der Radiographiemessung wird in Schritt 205 die Bestrahlung ggf. abgebrochen. Eventuell erfolgt hiernach sogar eine neue Bestrahlungsplanung.

Wenn die Radiographiemessung innerhalb vorbestimmter Grenzwerte liegt, kann in Schritt 204d - sofern notwendig - die Bestrahlung adaptiert werden. Anschließend wird in einem weiteren Schritt 204b die Bestrahlungsanlage wieder auf die Depositionsenergie E umgestellt, z.B. durch Hereinfahren der Energiemodulatorplatte 48' in den Strahlengang oder entsprechende Rotation des tortenartigen Energiemodulators 48 und durch entsprechende Wiederaufnahme des Scannvorgangs zur Dosisdeposition im Zielvolumen, welche dann wiederum in Schritt 204a fortgesetzt wird.

Somit wird die Radiographiemessung als Regelparameter innerhalb des Regelkreises 204a bis 204d eingesetzt.

Wenn der gesamte Bestrahlungsplan abgearbeitet ist, wird ebenfalls in Schritt 205 die Bestrahlung beendet. Die Anpassung der Bestrahlungsparameter beim Wechsel von der Radiographiephase 74 in die Depositionsphase 76 und umgekehrt in den Schritten 204b, erfolgt hier dadurch dass der Energiemodulators 48', 48 und die Scanneinrichtung 38 von der Steuereinrichtung 39 entsprechend angesteuert werden.

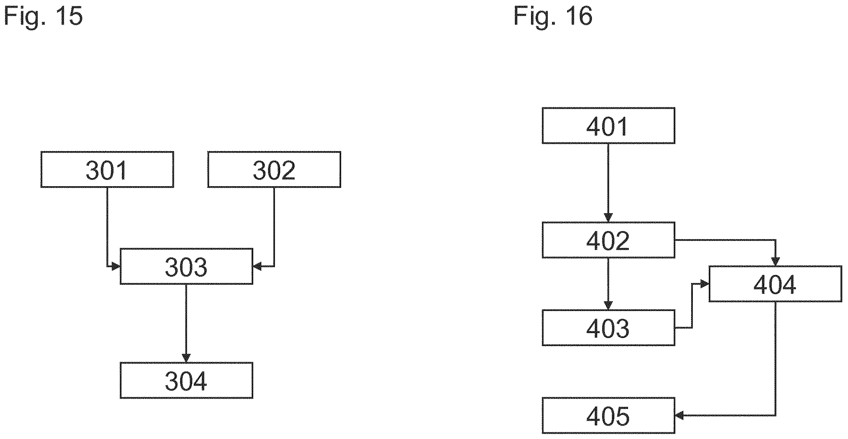

Fig. 15 zeigt ein Ablaufdiagramm zur Kombination der Radiographiemessung mit den Messergebnissen des Bewegungsmesssystems 54.

In Schritt 301 wird die Radiographiemessung mit dem Ionenradiographiedetektor 20 durchgeführt.

In Schritt 302 wird gleichzeitig und unabhängig hiervon die Bewegung des Zielvolumens mittels des Bewegungserfassungssystems 54, z.B. in Form einer Surrogatmessung erfasst.

In Schritt 303 vergleicht die Steuereinrichtung 39 die lonen-Radiographiemessung mit der Erwartung aus der Surrogatmessung und fällt in Ansprechen auf das Ergebnis dieses Vergleichs eine Entscheidung über den weiteren Verlauf bzw. einen evtl. Abbruch der Bestrahlung.

In Schritt 304 erfolgt bei Fortsetzung dann die Bestrahlung des Zielvolumens in einer Depositionsphase 76.

Im Übrigen entspricht der Verfahrensablauf demjenigen in Fig. 14.

Fig. 16 zeigt ein Ablaufschema einer Echtzeitauswertung mit DRRM.

In Schritt 401 wird für jede 4DCT-Phase eine DRRM berechnet. Dieser Schritt erfolgt insbesondere vor der Bestrahlung.

Die Box 402 symbolisiert die so berechneten DRRMs.

In Schritt 403 wird eine Radiographiemessung in einer Radiographiephase 74 durchgeführt.

In Schritt 404 wird, z.B. mittels der Steuereinrichtung 39, das Ergebnis der Radiographiemessung aus Schritt 403 mit der Erwartung aus der vorberechneten DRRM 402 verglichen. In Abhängigkeit des Ergebnisses dieses Vergleichs 404 wird in Schritt 405 ggf. die Bestrahlung adaptiert oder abgebrochen.

Es ist dem Fachmann ersichtlich, dass die vorstehend beschriebenen Ausführungsformen beispielhaft zu verstehen sind, und die Erfindung nicht auf diese beschränkt ist, sondern in vielfältiger Weise variiert werden kann, ohne den Schutzbereich der Ansprüche zu verlassen.

## Patentansprüche

1. Verfahren zur Bestrahlung eines Zielvolumens (12), welches nicht einem menschlichen oder tierischen Körper angehört, mit einem lonenstrahl (16, 18),
wobei die Bestrahlung des Zielvolumens (12) zeitlich in zumindest eine Radiographiephase (74) und zumindest eine Depositionsphase (76) unterteilt wird,
wobei die Energie (72) des lonenstrahls (16, 18) zwischen der zumindest einen Radiographiephase (74) und der zumindest einen Depositionsphase (76) zeitlich variiert wird, derart, dass
i) in der zumindest einen Radiographiephase (74) die Reichweite des lonenstrahls (16, 18) distal des Zielvolumens (12) liegt, so dass der lonenstrahl (16, 18) das Zielvolumen (12) durchdringt, und wobei mittels des lonenstrahls (16, 18) ein lonenradiogramm des Zielvolumens (12) aufgenommen wird, indem der lonenstrahl (16, 18) mit einem distal des Zielvolumens angeordneten lonenradiographiedetektor (20) detektiert wird und
ii) in der zumindest einen Depositionsphase (76) die Reichweite des lonenstrahls (16, 18) innerhalb des Zielvolumens (12) liegt, so dass der lonenstrahl (16, 18) in dem Zielvolumen (12) abgestoppt wird, um eine vorbestimmte Dosis in dem Zielvolumen (12) zu deponieren;
wobei in der zumindest einen oder der Mehrzahl von Radiographiephasen (74) ein lateral zweidimensional ortsaufgelöstes Ionenradiogramm aufgenommen wird, indem eine Vielzahl von Rasterpunkten des Zielvolumens (12) durchdrungen werden und die Reichweite des lonenstrahls nach dem Durchdringen des Zielvolumens für jeden der Rasterpunkte in dem Ionenradiogramm ermittelt wird; und
Erstellen einer zumindest zweidimensionalen Karte der Reichweite des lonenstrahls.

2. Bestrahlungsanlage (1) zur Bestrahlung eines Zielvolumens (12) mit einem lonenstrahl (16, 18), umfassend:
eine Beschleuniger- und Strahlführungseinrichtung (22, 24, 24', 26) zur Erzeugung und Beschleunigung eines lonenstrahls sowie zum Führen und Richten des lonenstrahls auf das Zielvolumen (12),
eine Steuereinrichtung (39) zur Steuerung der Bestrahlung,
eine Einrichtung (48, 48', 58a-58g) zum zeitlichen Variieren der Energie des lonenstrahls (16, 18) zwischen zumindest einer Radiographiephase (74) und zumindest einer Depositionsphase (76), mittels welcher
i) in der zumindest einen Radiographiephase (74) die Energie (72) des lonenstrahls (16, 18) auf eine Radiographieenergie eingestellt wird, bei welcher die Reichweite distal des Zielvolumens (12) liegt und der lonenstrahl (16, 18) das Zielvolumen (12) durchdringt,
ii) in der zumindest einen Depositionsphase (76) die Energie (72) des lonenstrahls (16, 18) auf eine Depositionsenergie eingestellt wird, bei welcher die Reichweite innerhalb des Zielvolumens (12) liegt und der lonenstrahl (16, 18) in dem Zielvolumen (12) abgestoppt wird, um eine vorbestimmte Dosis in dem Zielvolumen (12) zu deponieren,
einen distal des Zielvolumens (12) angeordneten Ionenradiographiedetektor (20) zum Aufnehmen von lonenradiogrammen des Zielvolumens (12), indem der das Zielvolumen (12) durchdringende lonenstrahl (16, 18) in der Radiographiephase (74) detektiert wird;
wobei der lonenradiographiedetektor (20) ein ortsauflösender Detektor ist, der jeweils ein lateral zweidimensional ortsaufgelöstes lonenradiogramm aufnimmt, indem in der zumindest einen oder der Mehrzahl von Radiographiephasen (74) jeweils eine Vielzahl von Rasterpunkten des Zielvolumens (12) von dem lonenstrahl durchdrungen werden,
ferner umfassend eine Recheneinrichtung zum Ermitteln der Reichweite des lonenstrahls (16, 18) nach dem Durchdringen des Zielvolumens (12) für jeden der Rasterpunkte in den lonenradiogrammen und zum Erstellen einer zumindest zweidimensionalen Karte der Reichweite des lonenstrahls nach dem Durchdringen des Zielvolumens (12).

3. Bestrahlungsanlage nach dem vorstehenden Anspruch,
wobei die Einrichtung zum zeitlichen Variieren der Energie (72) des lonenstrahls (16, 18) in einer wechselweisen Abfolge einer Mehrzahl von Radiographiephasen (74) und einer Mehrzahl von Depositionsphasen (76) die Energie des lonenstrahls (16, 18) hin und her schaltet, derart, dass zyklisch abwechselnd:
i) in den Radiographiephasen (74) die Energie (72) des lonenstrahls (16, 18) jeweils auf die Radiographieenergie eingestellt wird, so dass die Reichweite distal des Zielvolumens (12) liegt und der lonenstrahl (16, 18) das Zielvolumen (12) durchdringt,
ii) in den Depositionsphasen (76) die Energie des lonenstrahls auf die Depositionsenergie eingestellt wird, so dass die Reichweite innerhalb des Zielvolumens (12) liegt und der lonenstrahl (16, 18) in dem Zielvolumen (12) abgestoppt wird, um jeweils eine vorbestimmte Dosis in dem Zielvolumen (12) zu deponieren.

4. Bestrahlungsanlage nach Anspruch 3,
wobei die Steuereinrichtung (39) ausgebildet ist, die Bestrahlungsanlage (1) derart zu steuern, dass
in den Depositionsphasen (76) verschiedene Isoenergieschichten (13a-13i) des Zielvolumens (12) mit dem lonenstrahl (16, 18) angefahren werden, um jeweils eine vorbestimmte Dosis in den Isoenergieschichten zu deponieren und
zumindest vor der Bestrahlung zur Dosisdeposition jeder Isoenergieschicht eine Radiographiemessung mit dem Ionenradiographiedetektor (20) durchgeführt wird.

5. Bestrahlungsanlage nach einem der Ansprüche 2 bis 4,
wobei die Einrichtung zum zeitlichen Variieren der Energie (72) des lonenstrahls (16, 18) einen passiven Energiemodulator (48, 48') umfasst, welcher in der zumindest einen Depositionsphase (76) oder der Mehrzahl von Depositionsphasen (76) die Energie (72) des lonenstrahls (16, 18) von der höheren Radiographienergie auf die niedrigere Depositionsenergie herabsetzt.

6. Bestrahlungsanlage nach einem der Ansprüche 2 bis 5,
wobei die Steuereinrichtung (39) ausgebildet ist, die Bestrahlungsanlage (1) derart zu steuern, dass die Intensität (78) des lonenstrahls (16, 18) in der zumindest einen oder der Mehrzahl von Depositionsphasen (76) erheblich höher eingestellt ist, als in der zumindest einen oder der Mehrzahl von Radiographiephasen (74).

7. Bestrahlungsanlage nach einem der Ansprüche 2 bis 6,
umfassend eine Einrichtung zum Unterteilen der Bewegung eines sich während der Bestrahlung zyklisch bewegenden Zielvolumens (12) in mehrere Bewegungsphasen (12a-12k), wobei die Zeitdauer der zumindest einen Radiographiephase (74) oder der Mehrzahl von Radiographiephasen (74) jeweils nicht länger als die Zeitdauer der Bewegungsphasen ist; und/oder
umfassend eine Einrichtung zum aktivem Nachführen des lonenstrahls zur Kompensation der Bewegung des Zielvolumens (12), wobei die Steuereinrichtung (39) ausgebildet ist, das aktive Nachführen des lonenstrahls in Ansprechen auf die mittels des lonenradiographiedetektors (20) aufgenommenen lonenradiogramme zu steuern.

8. Bestrahlungsanlage nach einem der Ansprüche 2 bis 7,
umfassend eine Scann-Einrichtung (38) zum Scannen des lonenstrahls (18) über das Zielvolumen (12), wobei die Steuereinrichtung (39) ausgebildet ist, die Scanneinrichtung (38) so zu steuern, dass
der lonenstrahl (18) zur Dosisdeposition in der zumindest einen oder der Mehrzahl von Depositionsphasen (76) über das Zielvolumen (12) gescannt wird und
der lonenstrahl (18) in der zumindest einen oder der Mehrzahl von Radiographiephasen (74) zumindest über einen Teil der lateralen Fläche des Zielvolumens (12) gewobbelt wird.

9. Bestrahlungsanlage nach Anspruch 8, wobei die Steuereinrichtung (39) ausgebildet ist, die Scanneinrichtung (38) so zu steuern, dass der lonenstrahl (18) in der zumindest einen oder der Mehrzahl von Depositionsphasen (76) über das klinische Zielvolumen (CTV, ICRU 50) gescannt wird und der lonenstrahl (18) in der zumindest einen oder der Mehrzahl von Radiographiephasen (74) zumindest über einen über das klinische Zielvolumen (12) hinausgehenden Teil der lateralen Fläche des internen Zielvolumens (14, ITV, ICRU 62) gewobbelt wird.

10. Bestrahlungsanlage nach einem der Ansprüche 2 bis 9,
umfassend eine Recheneinrichtung, ausgebildet:
zur Durchführung einer Reichweiten-Simulationsrechnung, um simulierte Sollwerte für die Reichweite des lonenstrahls nach dem Durchdringen des Zielvolumens (12) in der zumindest einen oder der Mehrzahl von Radiographiephasen (74) zu berechnen,
zur Ermittlung der tatsächlichen Reichweite des lonenstrahls in Ansprechen auf Messwerte des lonenradiographiedetektors (20) nach dem Durchdringen des Zielvolumens (12) in der zumindest einen oder der Mahrzahl von Radiographiephasen (74) und
zum Vergleichen der tatsächlich ermittelten Reichweiten mit den simulierten Sollwerten.

11. Bestrahlungsanlage nach einem der Ansprüche 2 bis 10,
umfassend eine Recheneinrichtung, ausgebildet:
zur Durchführung einer Reichweiten-Simulationsrechnung für eine Vielzahl von Rasterpunkten, um eine mehrdimensionale simulierte Sollwert-Karte (DRRM) der Reichweite des lonenstrahls nach dem Durchdringen des Zielvolumens in der Radiographiephase (74) zu erstellen,
zur Ermittlung der tatsächliche Reichweiten des lonenstrahls nach dem Durchdringen des Zielvolumens (12) für eine Vielzahl von Rasterpunkten und zur Erstellung eines mehrdimensionalen Ionenradiogramms mit den jeweiligen tatsächlichen Reichweiten des lonenstrahls und
zum Vergleichen des lonenradiogramms mit der simulierten Sollwert-Karte.

12. Bestrahlungsanlage nach einem der Ansprüche 2 bis 11,
wobei ein internes oder externes Bewegungsmesssystem (54) zur Messung der Bewegung des Zielvolumens oder eines Bewegungssurrogats umfasst ist, und die Steuereinrichtung (39) ausgebildet ist:
Messergebnisse des Bewegungsmesssystems (54) aufzunehmen, und
lonenradiogramme des lonenradiographiedetektors (20) aufzunehmen, und
automatisch die Messergebnisse und die lonenradiogramme zu verknüpfen und die Bestrahlung in Ansprechen auf die verknüpften Daten zu steuern; und/oder den Wechsel zwischen den Radiographiephasen (74) und den Depositionsphasen (76) in Ansprechen auf die Messergebnisse des Bewegungsmesssystems (54) zu steuern.

13. Bestrahlungsanlage nach einem der Ansprüche 2 bis 12,
wobei die Bestrahlungsanlage (1) ausgebildet ist, das Zielvolumen (12) in der zumindest einen oder der Mehrzahl von Radiographiephasen (74) aus mehr als einer Richtung zu bestrahlen, um hiermit zumindest ein örtlich mehr als zweidimensionales Ionenradiogramm aufzunehmen.

## Claims

1. A method for irradiating a target volume (12) which does not belong to a human or animal body, using an ion beam (16, 18);
wherein the irradiation of the target volume (12) is subdivided in time into at least one radiography phase (74) and at least one deposition phase (76);
wherein the energy (72) of the ion beam (16, 18) is altered over time between the at least one radiography phase (74) and the at least one deposition phase (76) such that
i) in the at least one radiography phase (74), the range of the ion beam (16, 18) is distal with respect to the target volume (12), so that the ion beam (16, 18) passes through the target volume (12), and wherein the ion beam (16, 18) is used to capture an ion radiograph of the target volume (12) by detecting the ion beam (16, 18) using an ion radiography detector (20) that is disposed distal with respect to the target volume; and
ii) in the at least one deposition phase (76), the range of the ion beam (16, 18) is within the target volume (12), so that the ion beam (16, 18) is stopped in the target volume (12) to deposit a predetermined dose in the target volume (12);
wherein in the at least one or the plurality of radiography phases (74), a laterally two-dimensionally spatially resolved ion radiograph is acquired by having the ion beam passing through a multitude of grid points of the target volume (12) and the range of after passing it through a multitude of grid points of the target volume (12) and determining the range of the ion beam after it has passed through the target volume for each of the grid points in the ion radiograph; and
creating an at least two-dimensional map of the range of the ion beam.

2. An irradiation system (1) for irradiating a target volume (12) with an ion beam (16, 18), comprising:
an accelerator and beam guidance device (22, 24, 24', 26) for generating and accelerating an ion beam and for guiding and directing the ion beam to the target volume (12);
a control device (39) for controlling the irradiation;
a device (48, 48', 58a - 58g) for varying the energy of the ion beam (16, 18) over time between at least one radiography phase (74) and at least one deposition phase (76), whereby
i) in the at least one radiography phase (74), the energy (72) of the ion beam (16, 18) is set to a radiography energy having a range that is distal with respect to the target volume (12) and the ion beam (16, 18) passes through the target volume (12);
ii) in the at least one deposition phase (76), the energy (72) of the ion beam (16, 18) is set to a deposition energy having a range within the target volume (12) and the ion beam (16, 18) is stopped in the target volume (12) in order to deposit a predetermined dose in the target volume (12);
an ion radiography detector (20) disposed distally with respect to the target volume (12) for capturing ion radiographs of the target volume (12) by detecting the ion beam (16, 18) that has passes through the target volume (12) in the radiography phase (74);
wherein the ion radiography detector (20) is a spatially resolving detector that captures a laterally two-dimensionally spatially resolved ion radiograph at each time by having the ion beam passing through a multitude of grid points of the target volume (12) in each of the at least one or the plurality of radiography phases (74);
further comprising a computing device for determining the range of the ion beam (16, 18) after it has passed through the target volume (12) for each of the grid points in the ion radiographs and for creating an at least two-dimensional map of the range of the ion beam after it has passed through the target volume (12).

3. The irradiation system according to the preceding claim,
wherein the device for varying the energy (72) of the ion beam (16, 18) over time switches up and down the energy of the ion beam (16, 18) in an alternating sequence that includes a plurality of radiography phases (74) and a plurality of deposition phases (76), such that in a cyclically alternating manner:
i) in each of the radiography phases (74), the energy (72) of the ion beam (16, 18) is set to the radiography energy, such that the range thereof is distal with respect to the target volume (12) and the ion beam (16, 18) passes through the target volume (12);
ii) in each of the deposition phases (76), the energy of the ion beam is set to the deposition energy, such that the range thereof is within the target volume (12) and the ion beam (16, 18) is stopped in the target volume (12) in order to deposit a predetermined dose in the target volume (12).

4. The irradiation system according to claim 3,
wherein the control device (39) is configured to control the irradiation system (1) such that
in the deposition phases (76) different isoenergy layers (13a - 13i) of the target volume (12) are approached by the ion beam (16, 18) to deposit a respective predetermined dose in the isoenergy layer; and
wherein a radiography measurement is performed using the ion radiography detector (20) at least prior to the irradiation of each isoenergy layer for depositing a dose.

5. The irradiation system according to any one of claims 2 to 4,
wherein the device for varying the energy (72) of the ion beam (16, 18) over time comprises a passive energy modulator (48, 48') which, in the at least one deposition phase (76) or in the plurality of deposition phases (76), reduces the energy (72) of the ion beam (16, 18) from the higher radiography energy to the lower deposition energy.

6. The irradiation system according to any one of claims 2 to 5,
wherein the control device (39) is adapted to control the irradiation system (1) in a manner so that in the at least one or the plurality of deposition phases (76) the intensity (78) of the ion beam (16, 18) is set to be considerably higher than in the at least one or the plurality of radiography phases (74).

7. The irradiation system according to any one of claims 2 to 6,
comprising a device for dividing the movement of a target volume (12) that is cyclically moving during the irradiation into a plurality of movement phases (12a - 12k), wherein the duration of the at least one radiography phase (74) or of the plurality of radiography phases (74) is not longer than the duration of each of the movement phases; and/or
comprising a device for active ion beam tracking in order to compensate for the movement of the target volume (12), wherein the control device (39) is adapted to control the active ion beam tracking in response to the ion radiographs captured by the ion radiography detector (20).

8. The irradiation system according to any one of claims 2 to 7,
comprising a scanning device (38) for scanning the ion beam (18) across the target volume (12), wherein the control device (39) is adapted to control the scanning device so that
in the at least one or the plurality of deposition phases (76), the ion beam (18) is scanned across the target volume (12) to deposit a dose; and
in the at least one or the plurality of radiography phases (74), the ion beam (18) is wobbled across at least a portion of the lateral area of the target volume (12).

9. The irradiation system according to claim 8, wherein the control device (39) is adapted to control the scanning device (38) such that in the at least one or the plurality of deposition phases (76) the ion beam (18) is scanned across the clinical target volume (CTV, ICRU 50);
and in the at least one or the plurality of radiography phases (74) the ion beam (18) is wobbled across at least a portion of the lateral area of the internal target volume (14, ITV, ICRU 62) beyond the clinical target volume (12).

10. The irradiation system according to any one of claims 2 to 9, comprising a computing device that is configured to:
perform a range simulation calculation in order to calculate simulated target values for the range of the ion beam after it has passed through the target volume (12) in the at least one or the plurality of radiography phases (74);
determine the actual range of the ion beam after it has passed through the target volume (12) in the at least one or the plurality of radiography phases (74) in response to the measured values of the ion radiography detector (20); and
compare the ranges actually determined with the simulated target values.

11. The irradiation system according to any one of claims 2 to 10, comprising a computing device that is configured to:
perform a range simulation calculation for a multitude of grid points in order to create a multi-dimensional map of simulated target values (DRRM) of the range of the ion beam after it has passed through the target volume in the radiography phase (74);
determine the actual ranges of the ion beam after it has passed through the target volume (12) for a multitude of grid points, and to create a multi-dimensional ion radiograph with the respective actual ranges of the ion beam; and
compare the ion radiograph with the map of simulated target values.

12. The irradiation system according to any one of claims 2 to 11,
comprising an internal or external movement measuring system (54) for measuring the movement of the target volume or of a movement surrogate, and wherein the control device (39) is adapted:
to acquire measurement results of the movement measuring system (54); and
to acquire ion radiographs of the ion radiography detector (20); and
to automatically associate the measurement results and the ion radiographs and to control the irradiation in response to the associated data; and/or
to control the alternation between the radiography phases (74) and the deposition phases (76) in response to the measurement results of the movement measuring system (54).

13. The irradiation system according to any one of claims 2 to 12,
wherein the irradiation system (1) is configured to irradiate the target volume (12) from more than one direction in the at least one or the plurality of radiography phases (74) to thereby acquire at least one ion radiograph with more than two spatial dimensions.

## Revendications

1. Procédé d'irradiation d'un volume cible (12) qui ne fait pas partie d'un corps humain ou animal, comprenant un faisceau d'ions (16, 18),
selon lequel l'irradiation du volume cible (12) est séparée dans le temps en au moins une phase de radiographie (74) et au moins une phase de dépôt (76),
selon lequel l'énergie (72) du faisceau d'ions (16, 18) est variée dans le temps entre la phase de radiographie (74), au nombre d'au moins une, et la phase de dépôt (76), au nombre d'au moins une, de telle sorte que
i) lors de la phase de radiographie (74), au nombre d'au moins une, la portée du faisceau d'ions (16, 18) s'étend jusqu'à un emplacement distal par rapport au volume cible (12), de manière à ce que le faisceau d'ions (16, 18) traverse le volume cible (12), et le faisceau d'ions (16, 18) permettant d'enregistrer un radiogramme par ions du volume cible (12), par le fait que le faisceau d'ions (16, 18) est détecté à l'aide d'un détecteur de radiographie par ions (20) situé à un emplacement distal par rapport au volume cible, et
ii) lors de la phase de dépôt (76), au nombre d'au moins une, la portée du faisceau d'ions (16, 18) se situe à l'intérieur du volume cible (12), de manière à ce que le faisceau d'ions (16, 18) soit arrêté dans le volume cible (12) afin de déposer une dose prédéterminée dans le volume cible (12) ; sachant que lors de la phase de radiographie (74), au nombre d'au moins une, ou lors de la pluralité de phases de radiographie, on enregistre un radiogramme par ions à résolution spatiale latérale en deux dimensions, par le fait qu'une pluralité de points de trame du volume cible (12) sont traversés, et on détermine la portée du faisceau d'ions après avoir traversé le volume cible, pour chacun des points de trame dans le radiogramme par ions ; et
on réalise une carte en au moins deux dimensions de la portée du faisceau d'ions.

2. Installation d'irradiation (1) pour l'irradiation d'un volume cible (12) avec un faisceau d'ions (16, 18), comprenant :
un dispositif d'accélération et de guidage de faisceau (22, 24, 24', 26) destiné à générer et accélérer un faisceau d'ions, ainsi qu'à guider et diriger le faisceau d'ions sur le volume cible (12),
un dispositif de commande (39) destiné à contrôler l'irradiation,
un dispositif (48, 48', 58a-58g) destiné à faire varier dans le temps l'énergie du faisceau d'ions (16, 18), entre au moins une phase de radiographie (74) et au moins une phase de dépôt (76), à l'aide duquel
i) lors de la phase de radiographie (74), au nombre d'au moins une, l'énergie (72) du faisceau d'ions (16, 18) est réglée sur une énergie de radiographie avec laquelle la portée s'étend jusqu'à un emplacement distal par rapport au volume cible (12) et le faisceau d'ions (16, 18) traverse le volume cible (12),
ii) lors de la phase de dépôt (74), au nombre d'au moins une, l'énergie (72) du faisceau d'ions (16, 18) est réglée sur une énergie de dépôt avec laquelle la portée se situe à l'intérieur du volume cible (12) et le faisceau d'ions (16, 18) est arrêté dans le volume cible (12), afin de déposer une dose prédéterminée dans le volume cible (12),
un détecteur de radiographie par ions (20) qui est disposé à un emplacement distal par rapport au volume cible (12) et est destiné à enregistrer des radiogrammes par ions du volume cible (12), par le fait que le faisceau d'ions (16, 18) traversant le volume cible (12) est détecté lors de la phase de radiographie (74) ;
le détecteur de radiographie par ions (20) étant un détecteur à résolution spatiale qui enregistre respectivement un radiogramme par ions à résolution spatiale latérale en deux dimensions, par le fait qu'une pluralité de points de trame du volume cible (12) sont traversés par le faisceau d'ions lors de la phase de radiographie (74), au nombre d'au moins une, ou de la pluralité de phases de radiographie,
comprenant en outre un dispositif de calcul destiné à déterminer la portée du faisceau d'ions (16, 18) après qu'il a traversé le volume cible (12), pour chacun des points de trame dans les radiogrammes par ions, et à réaliser une carte, au moins en deux dimensions, de la portée du faisceau d'ions, après qu'il a traversé le volume cible (12).

3. Installation d'irradiation selon la revendication précédente,
dans laquelle le dispositif de variation dans le temps de l'énergie (72) du faisceau d'ions (16, 18) commute l'énergie du faisceau d'ions (16, 18) en une succession en alternance d'une pluralité de phases de radiographie (74) et d'une pluralité de phases de dépôt (76), de telle sorte que, dans une alternance cyclique :
i) lors des phases de radiographie (74), l'énergie (72) du faisceau d'ions (16, 18) soit réglée respectivement sur l'énergie de radiographie, de manière à ce que la portée s'étende jusqu'à un emplacement distal par rapport au volume cible (12) et le faisceau d'ions (16, 18) traverse le volume cible (12),
ii) lors des phases de dépôt (76), l'énergie du faisceau d'ions soit réglée sur l'énergie de dépôt, de manière à ce que la portée se situe à l'intérieur du volume cible (12) et le faisceau d'ions (16, 18) soit arrêté dans le volume cible (12), afin de déposer respectivement une dose prédéterminée dans le volume cible (12).

4. Installation d'irradiation selon la revendication 3,
dans laquelle le dispositif de commande (39) est conçu pour contrôler l'installation d'irradiation (1) de manière telle que
lors des phases de dépôt (76), différentes couches d'iso-énergie (13a-13i) du volume cible (12) soient ciblées avec le faisceau d'ions (16, 18), afin de déposer respectivement une dose prédéterminée dans les couches d'iso-énergie, et
au moins avant l'irradiation de chaque couche d'iso-énergie en vue du dépôt de dose, une mesure de radiographie soit effectuée à l'aide du détecteur de radiographie par ions (20).

5. Installation d'irradiation selon l'une des revendications 2 à 4,
dans laquelle le dispositif de variation dans le temps de l'énergie (72) du faisceau d'ions (16, 18) comprend un modulateur d'énergie passif (48, 48') qui, lors de la phase de dépôt (76), au nombre d'au moins une, ou de la pluralité de phases de dépôt, réduit l'énergie (72) du faisceau d'ions (16, 18) pour la faire passer de l'énergie de radiographie plus élevée à l'énergie de dépôt plus faible.

6. Installation d'irradiation selon l'une des revendications 2 à 5,
dans laquelle le dispositif de commande (39) est conçu pour contrôler l'installation d'irradiation (1) de manière telle que lors de la phase de dépôt (76), au nombre d'au moins une, ou de la pluralité de phases de dépôt, l'intensité (78) du faisceau d'ions (16, 18) soit réglée à une valeur sensiblement plus élevée que lors de la phase de radiographie (74), au nombre d'au moins une, ou de la pluralité de phases de radiographie.

7. Installation d'irradiation selon l'une des revendications 2 à 6,
comprenant un dispositif pour partager le mouvement d'un volume cible (12) qui se déplace de manière cyclique pendant l'irradiation, en plusieurs phases de mouvement (12a-12k), la durée de la phase de radiographie (74), au nombre d'au moins une, ou de la pluralité de phases de radiographie (74) ne dépassant respectivement pas la durée des phases de mouvement ; et/ou
comprenant un dispositif de guidage de poursuite actif du faisceau d'ions en vue de la compensation du mouvement du volume cible (12), le dispositif de commande (39) étant conçu pour contrôler le guidage de poursuite actif du faisceau d'ions en réponse aux radiogrammes par ions enregistrés à l'aide du détecteur de radiographie par ions (20).

8. Installation d'irradiation selon l'une des revendications 2 à 7,
comprenant un dispositif de balayage (38) destiné à balayer le faisceau d'ions (18) sur le volume cible (12), le dispositif de commande (39) étant conçu pour contrôler le dispositif de balayage (38) de manière telle que
le faisceau d'ions (18) balaie le volume cible (12) en vue du dépôt de dose dans la phase de dépôt (76), au nombre d'au moins une, ou dans la pluralité de phases de dépôt, et
le faisceau d'ions (18) soit soumis à une vobulation au moins sur une partie de la surface latérale du volume cible (12), lors de la phase de radiographie (74), au nombre d'au moins une, ou de la pluralité de phases de radiographie.

9. Installation d'irradiation selon la revendication 8, dans laquelle le dispositif de commande (39) est conçu pour contrôler le dispositif de balayage (38) de manière à ce que le faisceau d'ions (18) balaie le volume cible clinique (CTV, ICRU 50) lors de la phase de dépôt (76), au nombre d'au moins une, ou de la pluralité de phases de dépôt, et le faisceau d'ions (18) soit soumis à une vobulation au moins sur une partie de la surface latérale du volume cible interne (14, ITV, ICRU 62) s'étendant au-delà du volume cible clinique (12), lors de la phase de radiographie (74), au nombre d'au moins une, ou de la pluralité de phases de radiographie.

10. Installation d'irradiation selon l'une des revendications 2 à 9,
comprenant un dispositif de calcul, conçu
pour effectuer un calcul de simulation de portée, afin de calculer des valeurs de consigne simulées pour la portée du faisceau d'ions, après qu'il a traversé le volume cible (12), lors de la phase de radiographie (74), au nombre d'au moins une, ou de la pluralité de phases de radiographie,
pour déterminer la portée réelle du faisceau d'ions en réponse à des valeurs de mesure du détecteur de radiographie par ions (20), après la traversée du volume cible (12), lors de la phase de radiographie (74), au nombre d'au moins une, ou de la pluralité de phases de radiographie, et
pour comparer les portées effectivement déterminées avec les valeurs de consigne simulées.

11. Installation d'irradiation selon l'une des revendications 2 à 10,
comprenant un dispositif de calcul, conçu
pour effectuer un calcul de simulation de portée pour une pluralité de points de trame, afin de réaliser une carte de valeurs de consigne (DRRM) simulée, en plusieurs dimensions, de la portée du faisceau d'ions, après qu'il a traversé le volume cible lors de la phase de radiographie (74),
pour déterminer les portées réelles du faisceau d'ions, après qu'il a traversé le volume cible (12), pour une pluralité de points de trame, et pour réaliser un radiogramme par ions en plusieurs dimensions, avec les portées réelles respectives du faisceau d'ions, et
pour comparer le diagramme par ions avec la carte de valeurs de consigne simulée.

12. Installation d'irradiation selon l'une des revendications 2 à 11,
dans laquelle il est prévu un système de mesure de mouvement (54) interne ou externe, destiné à mesurer le mouvement du volume cible ou d'un substitut de mouvement, et le dispositif de commande (39) est conçu : pour enregistrer des résultats de mesure du système de mesure de mouvement (54), et pour enregistrer des radiogrammes par ions du détecteur de radiographie par ions (20), et pour combiner automatiquement les résultats de mesure et les diagrammes par ions, et
pour contrôler l'irradiation en réponse aux données combinées ; et/ou
pour commander le changement entre les phases de radiographie (74) et les phases de dépôt (76), en réponse aux résultats de mesure du système de mesure de mouvement (54).

13. Installation d'irradiation selon l'une des revendications 2 à 12,
où l'installation d'irradiation (1) est conçue pour irradier le volume cible (12) à partir de plus d'une direction, lors de la phase de radiographie (74), au nombre d'au moins une, ou de la pluralité de phases de radiographie, afin d'enregistrer ainsi au moins un radiogramme par ions présentant localement plus de deux dimensions.
